# EUROPEAN PATENT SPECIFICATION

(11) **EP 3 692 057 B1**
(45) Date of publication and mention of the grant of the patent: **07.06.2023**
(21) Application number: 18800781.9
(22) Date of filing: 03.10.2018
(51) Int. Cl.: C07K 14/71, C12N 5/00

(54) **MODIFIED EPIDERMAL GROWTH FACTOR RECEPTOR PEPTIDES FOR USE IN GENETICALLY-MODIFIED CELLS**
MODIFIZIERTE EPIDERMALE WACHSTUMSFAKTORREZEPTORPEPTIDE ZUR VERWENDUNG IN GENETISCH MODIFIZIERTEN ZELLEN
PEPTIDES DE RÉCEPTEUR DE FACTEUR DE CROISSANCE ÉPIDERMIQUE MODIFIÉS DESTINÉS À ÊTRE UTILISÉS DANS DES CELLULES GÉNÉTIQUEMENT MODIFIÉES

(30) Priority: 03.10.2017 US 201762567530 P
(43) Date of publication of application: 12.08.2020
(73) Proprietor: Precision BioSciences, Inc., Durham, NC 27701 (US)
(72) Inventor: JANTZ, Derek, Durham NC 27701 (US); SMITH, James, Jefferson, Morrisville NC 27560 (US); MACLEOD, Daniel, T., Durham NC 27705 (US)
(74) Representative: Grund, Martin
(86) International application number: PCT/US2018/054164
(87) International publication number: WO 2019/070856

(56) References cited:
- WO-A1-02/00876
- WO-A1-2013/123061
- WO-A1-2017/062439
- WO-A2-2011/056894
- WO-A2-2012/058588
- X. WANG ET AL: "A transgene-encoded cell surface polypeptide for selection, in vivo tracking, and ablation of engineered cells", BLOOD, vol. 118, no. 5, 4 August 2011 (2011-08-04), pages 1255-1263, XP055062819, ISSN: 0006-4971, DOI: 10.1182/blood-2011-02-337360

## Description

### FIELD OF THE INVENTION

The present disclosure relates to the field of molecular biology and recombinant nucleic acid technology. In particular, the present disclosure relates to modified epidermal growth factor receptor (EGFR) peptides. The present disclosure further relates to genetically-modified cells comprising the modified EGFR peptides and such cells for use in treatment of disease, such as cancer.

### BACKGROUND OF THE INVENTION

T cell adoptive immunotherapy is a promising approach for cancer treatment. This strategy utilizes isolated human T cells that have been genetically-modified to enhance their specificity for a specific tumor associated antigen. Genetic modification may involve the expression of a chimeric antigen receptor or an exogenous T cell receptor to graft antigen specificity onto the T cell. By contrast to exogenous T cell receptors, chimeric antigen receptors derive their specificity from the variable domains of a monoclonal antibody. Thus, T cells expressing chimeric antigen receptors (CAR T cells) induce tumor immunoreactivity in a major histocompatibility complex non-restricted manner. To date, T cell adoptive immunotherapy has been utilized as a clinical therapy for a number of cancers, including B cell malignancies (e.g., acute lymphoblastic leukemia (ALL), B cell non-Hodgkin lymphoma (NHL), and chronic lymphocytic leukemia), multiple myeloma, neuroblastoma, glioblastoma, advanced gliomas, ovarian cancer, mesothelioma, melanoma, and pancreatic cancer.

Despite its clinical benefits, T cell adoptive immunotherapy faces a number of challenges in both production and safety. The production of CAR T cells can require an initial transduction or transformation process, followed by an enrichment of CAR-positive T cells. Such enrichment steps can rely on positive selection using antibodies that bind to the CAR itself, or selection for the presence or absence of endogenous proteins which may have been up- or down-regulated along with expression of the CAR (*e.g*., T cell receptor knockout).

Various degrees of toxicity can be observed in patients following the administration of CAR T cells, including the development of cytokine release syndrome (CRS) and, in some cases, neurotoxicity. Allogeneic immunotherapy may have further safety concerns due to potential development of graft-versus-host-disease (GVHD), though knockout of certain cell-surface proteins (*e.g*., the T cell receptor, beta-2 microglobulin, etc.) may abrogate the development of GVHD.

Thus, to address both the production and safety issues of T cell adoptive immunotherapy, it would be advantageous to develop novel proteins which can be expressed by T cells and have a dual function: (1) use as a selection marker for CAR T cell enrichment *ex vivo;* and (2) use as a binding moiety *in vivo* for eradication of administered CAR T cells by, for example, a clinically-approved antibody.

Such dual-function proteins have been previously described by International Application Publication No. WO 2011/056894 (*i.e.,* the '894 publication), which discloses a truncated human epidermal growth factor receptor polypeptide, referred to as EGFRt. Specifically, the EGFRt polypeptide described by the '894 publication comprises the wild-type EGFR transmembrane domain, EGFR Domain IV, and EGFR Domain III which comprises the epitope for several clinical antibodies (*e.g*., cetuximab). Moreover, the EGFRt polypeptide lacks the membrane distal EGF-binding domains (*i.e*., EGFR Domain I and Domain II) and the cytoplasmic signaling tail which comprises the juxtamembrane domain and the tyrosine kinase-binding domain. Thus, the EGFRt protein is inert, and does not induce cell signaling after ligand or antibody binding. Similar polypeptides are disclosed in WO 2013/123061 and Wang et al., Blood 2011, 118(5):1255-1263.

The invention described herein represents an improvement over the prior art in a number of ways. The present inventors have developed modified EGFR peptides that retain the wild-type EGFR transmembrane domain and EGFR Domain III, but comprise a modified EGFR Domain IV. By comparison to the wild-type Domain IV sequence, the modified Domain IV sequences encompassed by the invention have been truncated at positions following each of the disulphide bonds in the domain. These truncated domains result in a smaller polypeptide and, consequently, a smaller nucleotide coding sequence. This is advantageous due to the size packaging restrictions of many adeno-associated viral (AAV) vectors, which are frequently used to introduce CAR coding sequences and other nucleic acid molecules into T cells. More notably, it was observed that modified EGFRs encompassed by the present invention are unexpectedly superior to the EGFRt polypeptide described by the '894 publication in their ability to bind an anti-EGFR antibody.

Further, the invention provides a modified EGFR that further comprises a CD34 epitope, such as the QBend10 epitope. Incorporation of this epitope into the modified EGFR allows for the isolation of CAR-positive T cells using a commercially-available, clinical grade (*i.e.,* GMP) anti-CD34 kit rather than undergoing the laborious task of developing a clinical grade anti-EGFR antibody suitable for *ex vivo* enrichment. As a result, incorporation of the CD34 epitope greatly simplifies the process of enriching cells *ex vivo* while having a viable epitope (*i.e.,* the modified EGFR) which can be targeted *in vivo* with a clinicallyavailable antibody (*i.e.* cetuximab) if it is necessary to destroy the CAR T cells after administration.

### SUMMARY OF THE INVENTION

The present disclosure provides modified epidermal growth factor receptor (EGFR) peptides that are useful for the selection of genetically-modified cells and for the depletion of administered genetically-modified cells *in vivo.* The present disclosure advances the art by providing EGFR peptides that exhibit enhanced binding by an anti-EGFR antibody and a small size to aid in viral vector packaging for delivery of the nucleotide coding sequence into cells. As described herein, modified EGFR peptides comprising an EGFR Domain III, an EGFR transmembrane domain, and a modified EGFR Domain IV, wherein said modified EGFR Domain IV is a truncated EGFR Domain IV, but lacking an EGFR Domain I, EGFR Domain II, an EGFR juxtamembrane domain, and/or an EGFR tyrosine kinase domain have increased binding properties to an anti-EGFR antibody (*e.g*., cetuximab) when compared to the EGFRt peptide described by the '894 publication (set forth herein as SEQ ID NO: 3). Also disclosed herein are genetically-modified cells that comprise a nucleic acid molecule encoding a presently disclosed modified EGFR peptide. These presently disclosed genetically-modified cells can further comprise a nucleotide sequence encoding a chimeric antigen receptor or an exogenous T cell receptor. Also provided herein are nucleic acid molecules, recombinant DNA constructs (*e.g*., plasmids), and viral vectors comprising a nucleic acid sequence encoding the modified EGFR peptides disclosed herein, populations of genetically-modified cells comprising the modified EGFR peptides, methods for producing and isolating such genetically-modified cells, and methods of administering compositions comprising the modified EGFR peptides to subjects in order to reduce the symptoms, progression, or occurrence of disease. In some embodiments, genetically-modified cells comprising the modified EGFR peptides disclosed herein are formulated as pharmaceutical compositions. They may be used, for example, as immunotherapy in the treatment of cancer.

Thus, in one aspect, the present disclosure provides a nucleic acid molecule comprising a nucleotide sequence encoding a modified human EGFR comprising an EGFR Domain III, a modified EGFR Domain IV, wherein said modified EGFR Domain IV is a truncated EGFR Domain IV, and an EGFR transmembrane domain, but not comprising an EGFR Domain I, an EGFR Domain II, an EGFR juxtamembrane domain, or an EGFR tyrosine kinase domain, and wherein the modified EGFR binds to an anti-EGFR antibody.

In various aspects of the present disclosure, the modified EGFR Domain IV of the modified human EGFR has at least 80%, at least 85%, at least 90%, at least 95%, at least 96%, at least 97%, at least 98%, or at least 99% sequence identity to an amino acid sequence set forth in any one of SEQ ID Nos: 5-9. In some embodiments, the modified EGFR peptide comprises a modified EGFR Domain IV having at least 90% sequence identity to an amino acid sequence of any one of SEQ ID NOs: 5-9. In specific embodiments, the modified EGFR peptide comprises a modified EGFR Domain IV having an amino acid sequence of any one of SEQ ID NOs: 5-9. In certain embodiments, the modified EGFR Domain IV is encoded by a nucleotide sequence having at least 80%, at least 85%, at least 90%, at least 95%, at least 96%, at least 97%, at least 98%, or at least 99% sequence identity to any one of SEQ ID NOs: 10-14. In certain embodiments, the modified EGFR Domain IV is encoded by a nucleotide sequence having at least 90% sequence identity to any one of SEQ ID NOs: 10-14. In some of these embodiments, the modified EGFR Domain IV is encoded by any one of the nucleotide sequences set forth in SEQ ID NOs: 10-14.

In various aspects of the present disclosure, the modified EGFR peptide has at least 80%, at least 85%, at least 90%, at least 95%, at least 96%, at least 97%, at least 98%, or at least 99% sequence identity to an amino acid sequence set forth in any one of SEQ ID NOs: 15-19. In some embodiments, the modified EGFR peptide comprises an amino acid sequence having at least 90% sequence identity to any one of SEQ ID NOs: 15-19. In certain embodiments, the modified EGFR peptide comprises an amino acid sequence of any one of SEQ ID NOs: 15-19. In certain embodiments, the modified EGFR peptide is encoded by a nucleotide sequence having at least 80%, at least 85%, at least 90%, at least 95%, at least 96%, at least 97%, at least 98%, or at least 99% sequence identity to any one of SEQ ID NOs: 20-24. In certain embodiments, the modified EGFR peptide is encoded by a nucleotide sequence having at least 90% sequence identity to any one of SEQ ID NOs: 20-24. In some of these embodiments, the modified EGFR peptide is encoded by any one of the nucleotide sequences set forth in SEQ ID NOs: 20-24.

In some embodiments, the modified human EGFR peptide binds to an anti-EGFR antibody selected from the group consisting of cetuximab, matuzumab, necitumumab, panitumumab, zalutumumab, and nimotuzumab. In particular embodiments, the modified human EGFR peptide binds to cetuximab.

The nucleic acid molecule encoding the presently disclosed modified EGFR peptides can further encode a signal sequence that directs the expression of the modified EGFR to the cell surface. In some of these embodiments, the signal sequence encoded by the nucleic acid molecule is a GMCSFR alpha chain signal sequence. In particular embodiments, the GMCSFR alpha chain signal sequence comprises SEQ ID NO: 27.

Along with a nucleotide sequence encoding the presently disclosed modified EGFR peptides, a nucleic acid molecule can further comprise a nucleotide sequence encoding a chimeric antigen receptor (CAR) or an exogenous T cell receptor (TCR). In some embodiments, the CAR or the exogenous TCR has specificity for a tumor antigen.

In some of these embodiments, the nucleic acid molecule comprises from the 5' to the 3' direction: the nucleotide sequence encoding the CAR or exogenous TCR, a 2A element or an IRES element, and the nucleotide sequence encoding the modified EGFR. In other embodiments, the nucleic acid molecule comprises in the 5' to 3' direction: the nucleotide sequence encoding the modified EGFR, a 2A element or an IRES element, and the nucleotide sequence encoding the CAR or exogenous TCR. In some of those embodiments wherein the nucleic acid molecule further comprises a nucleotide sequence encoding a CAR or exogenous TCR, the nucleic acid molecule further comprises a promoter that drives expression of the CAR or exogenous TCR.

In particular embodiments, the nucleic acid molecule comprising the nucleotide sequence encoding the presently disclosed modified EGFR peptides is an mRNA, a recombinant DNA construct, or a viral genome of a viral vector.

In certain embodiments, the nucleic acid molecule comprising the nucleotide sequence encoding the presently disclosed modified EGFR peptides further comprises a nucleotide sequence encoding a CD34 epitope. In some of these embodiments, the CD34 epitope is a QBend10 epitope. In certain embodiments, the QBend10 epitope comprises the amino acid sequence of SEQ ID NO: 28. In particular embodiments, the CD34 epitope is positioned at the C terminus or the N terminus of the EGFR Domain III, or at the C-terminus, the N-terminus, or within the modified EGFR Domain IV.

In another aspect, the present disclosure provides a recombinant DNA construct, wherein the recombinant DNA construct comprises the nucleic acid molecule described herein, which comprises a nucleotide sequence encoding a modified EGFR peptide comprising an EGFR Domain III, an EGFR transmembrane domain and a modified EGFR Domain IV, wherein said modified EGFR Domain IV is a truncated EGFR Domain IV, but lacks an EGFR Domain I, an EGFR Domain II, an EGFR juxtamembrane domain, and an EGFR tyrosine kinase domain, wherein the modified EGFR binds to an anti-EGFR antibody.

In some embodiments wherein the recombinant DNA construct comprises a modified EGFR peptide comprising a modified EGFR Domain IV, the modified EGFR Domain IV comprises an amino acid sequence having at least 90% sequence identity to any one of SEQ ID NOs: 5-9. In specific embodiments, the recombinant DNA construct comprises a nucleic acid molecule encoding a modified EGFR peptide comprising an EGFR Domain IV having an amino acid sequence set forth in any one of SEQ ID NOs: 5-9. In certain embodiments, the recombinant DNA construct comprises a nucleic acid molecule encoding a modified EGFR peptide comprising an amino acid sequence having at least 90% sequence identity to any one of SEQ ID NOs: 15-19 and 25. In some such embodiments, the recombinant DNA construct comprises a nucleic acid molecule encoding a modified EGFR peptide comprising an amino acid sequence set forth by any one of SEQ ID NOs: 15-19.

In certain embodiments, the recombinant DNA construct further comprises a nucleotide sequence encoding a CAR or exogenous TCR described herein.

In particular embodiments, the recombinant DNA construct further comprises a nucleotide sequence encoding a CD34 epitope described herein.

In some embodiments, the recombinant DNA construct encodes a viral vector. In some embodiments, the viral vector is a retroviral vector, a lentiviral vector, an adenoviral vector, or an adeno-associated virus (AAV) vector. In a specific embodiment, the viral vector is a recombinant AAV vector.

In some aspects, the present disclosure provides a viral vector comprising the nucleic acid molecule described herein which comprises a nucleotide sequence encoding a modified EGFR peptide comprising an EGFR Domain III, an EGFR transmembrane domain and a modified EGFR Domain IV, wherein said modified EGFR Domain IV is a truncated EGFR Domain IV, but lacks an EGFR Domain I, an EGFR Domain II, an EGFR juxtamembrane domain, and an EGFR tyrosine kinase domain, wherein the modified EGFR binds to an anti-EGFR antibody.

In some embodiments wherein the viral vector comprises a nucleotide sequence encoding a modified EGFR peptide comprising a modified EGFR Domain IV, the modified EGFR Domain IV comprises an amino acid sequence having at least 90% sequence identity to any one of SEQ ID NOs: 5-9. In specific embodiments, the viral vector comprises a nucleic acid molecule encoding a modified EGFR peptide comprising an EGFR Domain IV having an amino acid sequence set forth in any one of SEQ ID NOs: 5-9. In certain embodiments, the viral vector comprises a nucleic acid molecule encoding a modified EGFR peptide comprising an amino acid sequence having at least 90% sequence identity to any one of SEQ ID NOs: 15-19 and 25. In some such embodiments, the viral vector comprises a nucleic acid molecule encoding a modified EGFR peptide comprising an amino acid sequence set forth by any one of SEQ ID NOs: 15-19.

In some such embodiments, the viral vector is a retroviral vector, a lentiviral vector, an adenoviral vector, or an adeno-associated virus (AAV) vector. In a specific embodiment, the viral vector is a recombinant AAV vector.

In certain embodiments, the viral vector further comprises a nucleotide sequence encoding a CAR or exogenous TCR described herein.

In other embodiments, the viral vector further comprises a nucleotide sequence encoding a CD34 epitope described herein.

In another aspect, the present disclosure provides a genetically-modified cell, wherein the genetically-modified cell comprises in its genome the nucleic acid molecule described herein which comprises a nucleotide sequence encoding a modified EGFR peptide comprising an EGFR Domain III, an EGFR transmembrane domain and a modified EGFR Domain IV, wherein said modified EGFR Domain IV is a truncated EGFR Domain IV, but lacks an EGFR Domain I, an EGFR Domain II, an EGFR juxtamembrane domain, and an EGFR tyrosine kinase domain, wherein the modified EGFR binds to an anti-EGFR antibody.

In some embodiments wherein the genetically-modified cell comprises a modified EGFR peptide comprising a modified EGFR Domain IV, the modified EGFR Domain IV comprises an amino acid sequence having at least 90% sequence identity to any one of SEQ ID NOs: 5-9. In specific embodiments, the genetically-modified cell comprises a nucleic acid molecule encoding a modified EGFR peptide comprising an EGFR Domain IV having an amino acid sequence set forth in any one of SEQ ID NOs: 5-9. In certain embodiments, the genetically-modified cell comprises a nucleic acid molecule encoding a modified EGFR peptide comprising an amino acid sequence having at least 90% sequence identity to any one of SEQ ID NOs: 15-19 and 25. In some such embodiments, the genetically-modified cell comprises a nucleic acid molecule encoding a modified EGFR peptide comprising an amino acid sequence set forth by any one of SEQ ID NOs: 15-19.

In yet another aspect, the present disclosure provides a genetically-modified cell comprising in its genome a nucleic acid molecule disclosed herein, wherein the nucleic acid molecule comprises a nucleotide sequence encoding a modified EGFR receptor and a nucleotide sequence encoding a CD34 epitope described herein.

In some of these embodiments, the genetically-modified cell comprises a CD34 epitope that is a QBend10 epitope. In particular embodiments, the QBend0 epitope comprises the amino acid sequence of SEQ ID NO: 28. In particular embodiments, the genetically-modified cell comprises a CD34 epitope that is positioned at the C-terminus or the N-terminus of the EGFR Domain III, or at the C-terminus, the N-terminus, or within the modified EGFR Domain IV.

In certain embodiments, the genetically-modified cell further expresses a chimeric antigen receptor (CAR) or an exogenous T cell receptor (TCR) described herein. In particular embodiments, the CAR or exogenous TCR has specificity for a tumor antigen.

In some embodiments, the nucleic acid molecule encoding a modified EGFR peptide is positioned within the endogenous TCR alpha gene of the genetically-modified cell. In some of these embodiments, the nucleic molecule encoding a modified EGFR peptide is positioned within the endogenous TCR alpha constant region gene. In particular embodiments, the nucleic acid molecule is positioned within an intron 5' upstream of exon 1 of the TCRα constant region gene.

In particular aspects, the genetically-modified cell comprising a nucleic acid molecule disclosed herein is a genetically-modified human T cell or a cell derived therefrom.

In certain embodiments, the genetically-modified cell is a genetically-modified human T cell which expresses a modified EGFR peptide disclosed herein, and the genetically-modified human T cell expresses a CAR or an exogenous TCR. In particular embodiments, the CAR or exogenous TCR has specificity for a tumor antigen.

In certain embodiments, the genetically-modified cell is a genetically-modified human T cell which expresses a modified EGFR peptide disclosed herein that comprises a QBend10 epitope, and the genetically-modified human T cell expresses a CAR or an exogenous TCR. In particular embodiments, the CAR or exogenous TCR has specificity for a tumor antigen.

In still another aspect, the present disclosure provides a population of genetically-modified cells comprising a plurality of genetically-modified cells described herein that express a modified EGFR as disclosed herein, and wherein the genetically-modified cells comprise in its genome the nucleic acid molecule described herein which comprises a nucleotide sequence encoding a modified EGFR peptide comprising an EGFR Domain III, an EGFR transmembrane domain and a modified EGFR Domain IV, wherein said modified EGFR Domain IV is a truncated EGFR Domain IV, but lacks an EGFR Domain I, an EGFR Domain II, an EGFR juxtamembrane domain, and an EGFR tyrosine kinase domain, wherein the modified EGFR binds to an anti-EGFR antibody.

In some embodiments wherein the genetically-modified cells within the population comprise a modified EGFR peptide comprising a modified EGFR Domain IV, the modified EGFR Domain IV comprises an amino acid sequence having at least 90% sequence identity to any one of SEQ ID NOs: 5-9. In specific embodiments, the genetically-modified cells within the population comprise a nucleic acid molecule encoding a modified EGFR peptide comprising an EGFR Domain IV having an amino acid sequence set forth in any one of SEQ ID NOs: 5-9. In certain embodiments, the genetically-modified cells within the population of cells comprise a nucleic acid molecule encoding a modified EGFR peptide comprising an amino acid sequence having at least 90% sequence identity to any one of SEQ ID NOs: 15-19 and 25. In some such embodiments, the genetically-modified cells within the population comprise a nucleic acid molecule encoding a modified EGFR peptide comprising an amino acid sequence set forth by any one of SEQ ID NOs: 15-19.

In some of these embodiments, the genetically-modified cells within the population express a modified EGFR described herein that comprises a CD34 epitope described herein. In some of these embodiments, the genetically-modified cells within the population comprise a CD34 epitope that is a QBend10 epitope comprising the amino acid sequence of SEQ ID NO: 28. In particular embodiments, the genetically-modified cells within the population comprise a CD34 epitope that is positioned at the C-terminus or N-terminus of the EGFR Domain III, or at the C-terminus, the N-terminus, or within the modified EGFR Domain IV. In specific embodiments, the genetically-modified cells disclosed herein comprise a Qbend10 epitope within a modified EGFR Domain IV, such as at the C-terminus, the N-terminus, or anywhere within the modified EGFR Domain IV.

In some embodiments, the genetically-modified cells within the population of cells further express a CAR or an exogenous TCR. In particular embodiments, the CAR or the exogenous TCR has specificity for a tumor antigen. In certain embodiments, the genetically-modified cells within the population of cells are genetically-modified human T cells or cells derived therefrom. In particular embodiments, at least 50%, at least 60%, at least 70%, at least 80%, at least 85%, at least 90%, at least 95%, or up to 100% of the genetically-modified cells in the population express a modified EGFR and a CAR or an exogenous TCR.

The present disclosure further provides a method for producing a genetically-modified cell expressing a modified EGFR described herein, the method comprising: (a) introducing into a cell at least one nucleic acid molecule described herein encoding at least one modified EGFR peptide described herein; and (b) introducing into the cell: (i) a second nucleic acid molecule encoding an engineered nuclease that is expressed in the cell; or (ii) an engineered nuclease protein, wherein the engineered nuclease recognizes and cleaves a recognition sequence in the genome of the cell to produce a cleavage site, and wherein the nucleic acid molecule encoding the modified EGFR is inserted into the genome of the cell at the cleavage site. The nucleic acid molecule encoding a modified EGFR may further encode a CD34 epitope described herein and/or a CAR or exogenous TCR described herein.

The engineered nuclease may be an engineered meganuclease, a recombinant zinc-finger nuclease (ZFN), a recombinant transcription activator-like effector nuclease (TALEN), a CRISPR nuclease, or a megaTAL nuclease.

The engineered nuclease may be an engineered meganuclease.

The nucleic acid molecule encoding a modified EGFR may further comprise sequences homologous to sequences flanking the cleavage site such that the nucleic acid molecule is inserted into the genome of the cell at the cleavage site by homologous recombination.

The nucleic acid molecule encoding a modified EGFR may lack substantial homology to the cleavage site such that the nucleic acid molecule is inserted into the genome of the cell by non-homologous end joining.

The cleavage site may be positioned within the T cell receptor alpha (TCRα) gene. The cleavage site may be positioned within the TCRα constant region gene. The cleavage site may be positioned within an intron 5' upstream of exon 1 of the endogenous TCRα constant region gene.

The nucleic acid molecule encoding the modified EGFR that is introduced into the cell may be an mRNA described herein. The nucleic acid molecule encoding the modified EGFR that is introduced into the cell may be a recombinant DNA construct described herein. The nucleic acid molecule encoding the modified EGFR that is introduced into the cell may be a viral vector described herein.

When a second nucleic acid molecule encoding an engineered nuclease is introduced into the cell, the second nucleic acid molecule can be an mRNA. Alternatively, when a second nucleic acid molecule encoding an engineered nuclease is introduced into the cell, the second nucleic acid molecule can be a recombinant DNA construct or a viral vector.

The method may comprise producing a genetically-modified eukaryotic cell. The method may comprise producing a genetically-modified human T cell or a cell derived therefrom.

In another aspect, the present disclosure also provides a genetically-modified cell prepared by any of the methods for producing a genetically-modified cell described herein.

In another aspect, the present disclosure provides a pharmaceutical composition comprising a pharmaceutically-acceptable carrier and genetically-modified cells described herein which comprise a nucleotide sequence encoding a modified EGFR peptide comprising an EGFR Domain III, an EGFR transmembrane domain and a modified EGFR Domain IV, wherein said modified EGFR Domain IV is a truncated EGFR Domain IV, but lacking an EGFR Domain I, an EGFR Domain II, an EGFR juxtamembrane domain, and an EGFR tyrosine kinase domain, wherein the modified EGFR binds to an anti-EGFR antibody.

In some embodiments wherein the genetically-modified cells comprise a modified EGFR peptide comprising a modified EGFR Domain IV, the modified EGFR Domain IV comprises an amino acid sequence having at least 90% sequence identity to any one of SEQ ID NOs: 5-9. In specific embodiments, the genetically-modified cells comprise a nucleic acid molecule encoding a modified EGFR peptide comprising an EGFR Domain IV having an amino acid sequence set forth in any one of SEQ ID NOs: 5-9. In certain embodiments, the genetically-modified cells comprise a nucleic acid molecule encoding a modified EGFR peptide comprising an amino acid sequence having at least 90% sequence identity to any one of SEQ ID NOs: 15-19. In some such embodiments, the genetically-modified cells comprise a nucleic acid molecule encoding a modified EGFR peptide comprising an amino acid sequence set forth by any one of SEQ ID NOs: 15-19. In certain embodiments, the genetically-modified cells further express a CD34 epitope and/or a CAR or exogenous TCR.

In particular embodiments, the pharmaceutical composition comprises genetically-modified human T cells as described herein, or cells derived therefrom.

In another aspect, the present disclosure provides a method for isolating genetically-modified cells described herein that expresses a modified EGFR peptide disclosed herein, wherein the method comprises: (a) contacting the genetically-modified cells with an anti-EGFR antibody; and (b) selecting the genetically-modified cells which are bound to the anti-EGFR antibody.

In certain embodiments of the method, the genetically-modified cells that are isolated comprise a nucleotide sequence encoding a modified EGFR peptide comprising an EGFR Domain III, an EGFR transmembrane domain and a modified EGFR Domain IV, wherein said modified EGFR Domain IV is a truncated EGFR Domain IV, but lacking an EGFR Domain I, an EGFR Domain II, an EGFR juxtamembrane domain, and an EGFR tyrosine kinase domain, wherein the modified EGFR binds to an anti-EGFR antibody.

In some embodiments of the method, the genetically-modified cells that are isolated using the presently disclosed methods comprise a modified EGFR peptide comprising a modified EGFR Domain IV, the modified EGFR Domain IV comprises an amino acid sequence having at least 90% sequence identity to any one of SEQ ID NOs: 5-9. In specific embodiments, the genetically-modified cells that are isolated using the methods described herein comprise a nucleic acid molecule encoding a modified EGFR peptide comprising an EGFR Domain IV having an amino acid sequence set forth in any one of SEQ ID NOs: 5-9. In certain embodiments, the genetically-modified cells isolated using the presently disclosed methods comprise a nucleic acid molecule encoding a modified EGFR peptide comprising an amino acid sequence having at least 90% sequence identity to any one of SEQ ID NOs: 15-19. In some such embodiments, the genetically-modified cells comprise a nucleic acid molecule encoding a modified EGFR peptide comprising an amino acid sequence set forth by any one of SEQ ID NOs: 15-19.

In certain embodiments of the method, the genetically-modified cells further express a CD34 epitope described herein and/or a CAR or exogenous TCR described herein. In some of these embodiments, the anti-EGFR antibody is biotinylated, wherein the selection can be performed using anti-biotin or streptavidin-coated particles. In some of these embodiments, the anti-EGFR antibody is cetuximab, matuzumab, necitumumab, panitumumab, zalutumumab, or nimotuzumab.

In other embodiments of the method, the genetically-modified cell further expresses a CD34 epitope described herein, and the method of isolating the genetically-modified cells that express the modified EGFR comprises: (a) contacting the genetically-modified cells with an anti-CD34 antibody; and (b) selecting the genetically-modified cells that are bound to the anti-CD34 antibody. In some of these embodiments, the anti-CD34 antibody is conjugated to a particle, such as a magnetic bead. In those embodiments wherein the CD34 epitope is a QBend10 epitope, the anti-CD34 antibody used for isolation is the QBend10 antibody.

Further disclosed is a method of administering genetically-modified cells to a subject, wherein the genetically-modified cells are those described herein which express a modified EGFR peptide described herein.

The genetically-modified cells, or populations thereof, administered to a subject comprise a nucleotide sequence encoding a modified EGFR peptide comprising an EGFR Domain III, an EGFR transmembrane domain and a modified EGFR Domain IV, wherein said modified EGFR Domain IV is a truncated EGFR Domain IV, but lacking an EGFR Domain I, an EGFR Domain II, an EGFR juxtamembrane domain, and an EGFR tyrosine kinase domain, wherein the modified EGFR binds to an anti-EGFR antibody.

When the genetically-modified cells are administered to a subject, the cells may comprise a modified EGFR peptide comprising a modified EGFR Domain IV, wherein the modified EGFR Domain IV comprises an amino acid sequence having at least 90% sequence identity to any one of SEQ ID NOs: 5-9. The genetically-modified cells administered to a subject may comprise a nucleic acid molecule encoding a modified EGFR peptide comprising an EGFR Domain IV having an amino acid sequence set forth in any one of SEQ ID NOs: 5-9. The genetically-modified cells administered to a subject may comprise a nucleic acid molecule encoding a modified EGFR peptide comprising an amino acid sequence having at least 90% sequence identity to any one of SEQ ID NOs: 15-19. The genetically-modified cells administered to a subject may comprise a nucleic acid molecule encoding a modified EGFR peptide comprising an amino acid sequence set forth by any one of SEQ ID NOs: 15-19.

The genetically-modified cells administered to a subject may further express a CD34 epitope described herein.

The genetically-modified cells administered to a subject may further express a CAR or an exogenous TCR described herein.

The subject administered the genetically-modified cells may have a disease, and the genetically-modified cells may further express a CAR or an exogenous TCR that has specificity for a marker for the disease.

The CAR or exogenous TCR may have specificity for a cancer or tumor-specific antigen, and the genetically-modified cells may be useful in methods of cancer immunotherapy.

The cancer may be a carcinoma, lymphoma, sarcoma, blastoma, or leukemia. The cancer may be a cancer of B-cell origin (*e.g*., B-lineage acute lymphoblastic leukemia, B-cell chronic lymphocytic leukemia, B-cell lymphoma, diffuse large B cell lymphoma, pre-B ALL (pediatric indication), mantle cell lymphoma, follicular lymphoma, marginal zone lymphoma, Burkitt's lymphoma, and B-cell non-Hodgkin's lymphoma), breast cancer, gastric cancer, neuroblastoma, osteosarcoma, lung cancer, melanoma, prostate cancer, colon cancer, renal cell carcinoma, ovarian cancer, rhabdomyosarcoma, leukemia, and Hodgkin's lymphoma.

Further disclosed are genetically-modified cells, or populations thereof, described herein that express a modified EGFR peptide described herein for use as a medicament.

The genetically-modified cells or populations thereof for use as a medicament can comprise a nucleotide sequence encoding a modified EGFR peptide comprising an EGFR Domain III, an EGFR transmembrane domain and a modified EGFR Domain IV, wherein said modified EGFR Domain IV is a truncated EGFR Domain IV, but lacking an EGFR Domain I, an EGFR Domain II, an EGFR juxtamembrane domain, and an EGFR tyrosine kinase domain, wherein the modified EGFR binds to an anti-EGFR antibody.

When the genetically-modified cells used as a medicament comprise a modified EGFR peptide comprising a modified EGFR Domain IV, the modified EGFR Domain IV may comprise an amino acid sequence having at least 90% sequence identity to any one of SEQ ID NOs: 5-9. The genetically-modified cells used as a medicament may comprise a nucleic acid molecule encoding a modified EGFR peptide comprising an EGFR Domain IV having an amino acid sequence set forth in any one of SEQ ID NOs: 5-9. The genetically-modified cells used as a medicament may comprise a nucleic acid molecule encoding a modified EGFR peptide comprising an amino acid sequence having at least 90% sequence identity to any one of SEQ ID NOs: 15-19. The genetically-modified cells used as a medicament may comprise a nucleic acid molecule encoding a modified EGFR peptide comprising an amino acid sequence set forth by any one of SEQ ID NOs: 15-19.

The genetically-modified cells used as a medicament may further express a CD34 epitope described herein.

The genetically-modified cells used as a medicament may further express a CAR or an exogenous TCR described herein.

Further disclosed are genetically-modified cells or populations thereof described herein that express a modified EGFR peptide described herein for use in treating a disease in a subject in need thereof.

The genetically-modified cells, or populations thereof, for use in treating a disease comprise a nucleotide sequence encoding a modified EGFR peptide described herein comprising an EGFR Domain III, an EGFR transmembrane domain, and a modified EGFR Domain IV, wherein said modified EGFR Domain IV is a truncated EGFR Domain IV, but lacking an EGFR Domain I, an EGFR Domain II, an EGFR juxtamembrane domain, and an EGFR tyrosine kinase domain, wherein the modified EGFR binds to an anti-EGFR antibody.

When the genetically-modified cells for treating a disease comprise a modified EGFR peptide comprising a modified EGFR Domain IV, the modified EGFR Domain IV may comprise an amino acid sequence having at least 90% sequence identity to any one of SEQ ID NOs: 5-9. The genetically-modified cells for treating a disease may comprise a nucleic acid molecule encoding a modified EGFR peptide comprising an EGFR Domain IV having an amino acid sequence set forth in any one of SEQ ID NOs: 5-9. The genetically-modified cells for treating a disease may comprise a nucleic acid molecule encoding a modified EGFR peptide comprising an amino acid sequence having at least 90% sequence identity to any one of SEQ ID NOs: 15-19. The genetically-modified cells for treating a disease may comprise a nucleic acid molecule encoding a modified EGFR peptide comprising an amino acid sequence set forth by any one of SEQ ID NOs: 15-19.

The genetically-modified cells for treating a disease may further express a CD34 epitope described herein.

The genetically-modified cells for treating a disease may further express a CAR or exogenous TCR described herein. The CAR or exogenous TCR may have specificity for a tumor antigen and is for use in treating cancer in subjects in need thereof.

Further disclosed is a method for depleting a genetically-modified cell described herein within a subject, wherein the method comprises administering to the subject an anti-EGFR antibody that binds to the modified EGFR peptide and has complement-dependent cytotoxicity (CDC) or antibody-dependent cell-mediated cytotoxicity (ADCC) activity.

The anti-EGFR antibody that is administered to the subject may be cetuximab, matuzumab, necitumumab, panitumumab, zalutumumab, or nimotuzumab.

Further disclosed is a method for depleting a genetically-modified cell that expresses a modified EGFR peptide described herein and a CD34 epitope, wherein the method comprises administering to the subject an anti-CD34 antibody that binds to the CD34 epitope and has CDC or ADCC activity. When the genetically-modified cells express a QBend10 epitope comprising an amino acid sequence of SEQ ID NO: 28, the anti-CD34 antibody that is administered to the subject may be a QBend10 anti-CD34 antibody.

The genetically-modified cells that are depleted using these methods comprise a nucleotide sequence encoding a modified EGFR peptide comprising an EGFR Domain III, an EGFR transmembrane domain and a modified EGFR Domain IV, wherein said modified EGFR Domain IV is a truncated EGFR Domain IV, but lacking an EGFR Domain I, an EGFR Domain II, an EGFR juxtamembrane domain, and an EGFR tyrosine kinase domain, wherein the modified EGFR binds to an anti-EGFR antibody.

The depleted genetically-modified cells may comprise a modified EGFR peptide comprising a modified EGFR Domain IV, the modified EGFR Domain IV comprises an amino acid sequence having at least 90% sequence identity to any one of SEQ ID NOs: 5-9. The genetically-modified cells that are depleted using the presently disclosed methods may comprise a nucleic acid molecule encoding a modified EGFR peptide comprising an EGFR Domain IV having an amino acid sequence set forth in any one of SEQ ID NOs: 5-9. The depleted genetically-modified cells may comprise a nucleic acid molecule encoding a modified EGFR peptide comprising an amino acid sequence having at least 90% sequence identity to any one of SEQ ID NOs: 15-19. The genetically-modified cells that are depleted using the presently disclosed methods may comprise a nucleic acid molecule encoding a modified EGFR peptide comprising an amino acid sequence set forth by any one of SEQ ID NOs: 15-19.

The depleted genetically-modified cells may further express a CAR or an exogenous TCR. The CAR or exogenous TCR may have specificity for a tumor antigen.

The foregoing and other aspects and embodiments of the present invention can be more fully understood by reference to the following detailed description and claims. Certain features of the invention, which are, for clarity, described in the context of separate embodiments, may also be provided in combination in a single embodiment. All combinations of the embodiments are specifically embraced by the present invention and are disclosed herein just as if each and every combination was individually and explicitly disclosed. Conversely, various features of the invention, which are, for brevity, described in the context of a single embodiment, may also be provided separately or in any suitable sub-combination. All sub-combinations of features listed in the embodiments are also specifically embraced by the present invention and are disclosed herein just as if each and every such sub-combination was individually and explicitly disclosed herein. Embodiments of each aspect of the present invention disclosed herein apply to each other aspect of the invention *mutatis mutandis.* Notwithstanding the above, subject-matter which is not comprised by the scope of the claims does not form part of the present invention.

### BRIEF DESCRIPTION OF THE FIGURES

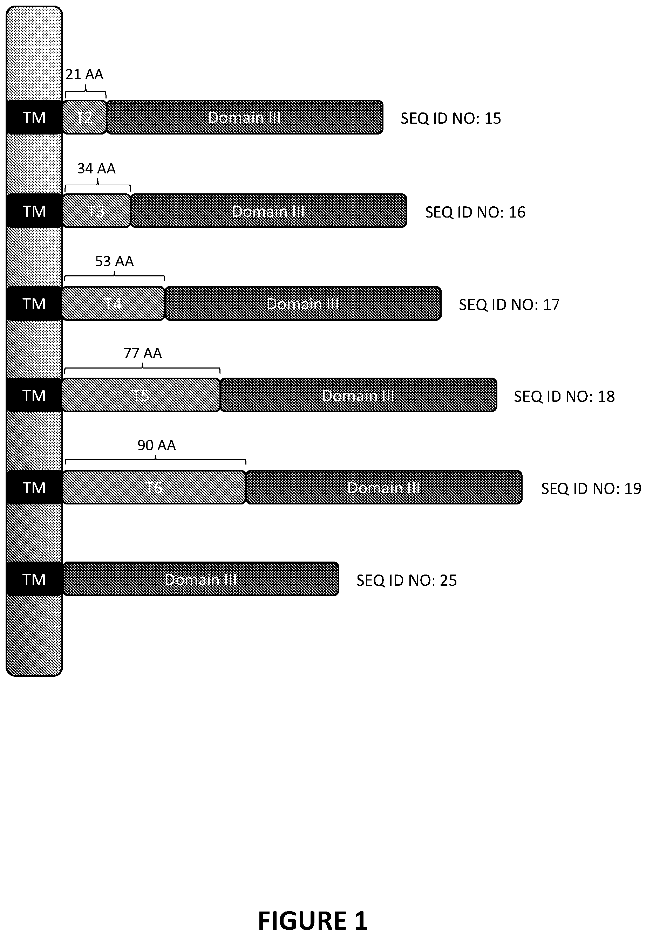

Figure 1 shows a schematic of modified human EGFR peptides described herein. Each modified EGFR peptide shares a wild-type human EGFR transmembrane domain and a wild-type EGFR Domain III, with an intervening modified EGFR Domain IV (SEQ ID NOs: 15-19) or an absent EGFR Domain IV (SEQ ID NO: 25).

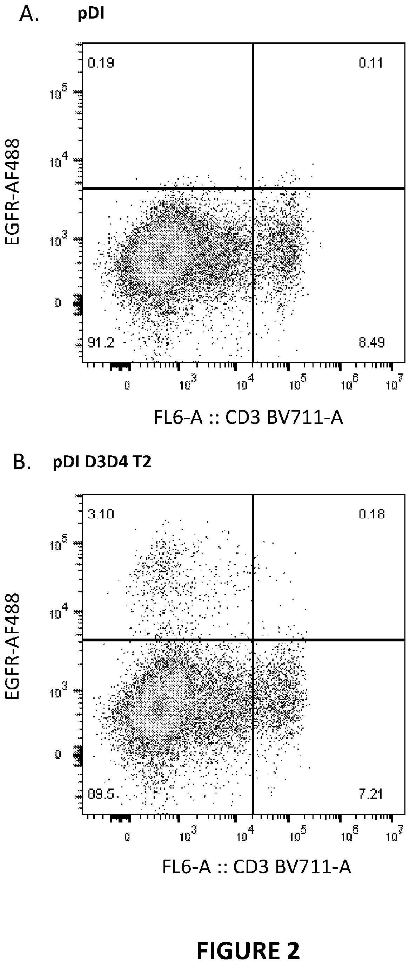

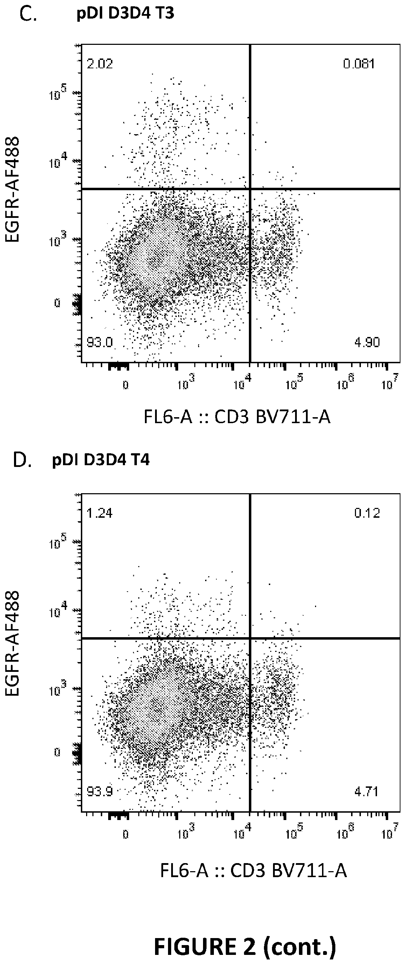

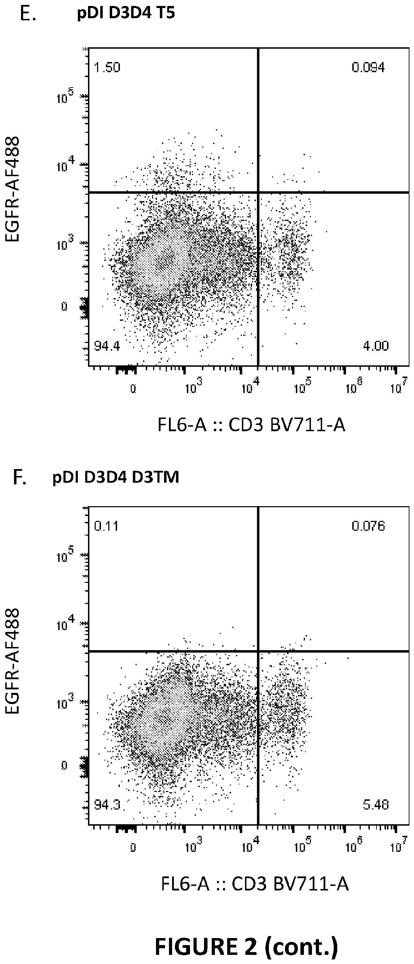

Figure 2 shows a screen for binding of modified human EGFR peptides to cetuximab. Modified human EGFR peptides (T2-T5 variants set forth in SEQ ID NOs: 15-18) were expressed in human T cells following targeted insertion of coding sequences into exon 1 of the T cell receptor alpha constant region gene. The ability of each modified EGFR peptide to bind to cetuximab was determined by staining and flow cytometry. Figure 2A shows binding data for the pDI vector control. Figure 2B shows binding data for the T2 EGFR variant (SEQ ID NO: 15). Figure 2C shows binding data for the T3 EGFR variant (SEQ ID NO: 16). Figure 2D shows binding data for the T4 EGFR variant (SEQ ID NO: 17). Figure 2E shows binding data for the T5 EGFR variant (SEQ ID NO: 18). Figure 2F shows binding data for the EGFR variant lacking a Domain IV (SEQ ID NO: 25).

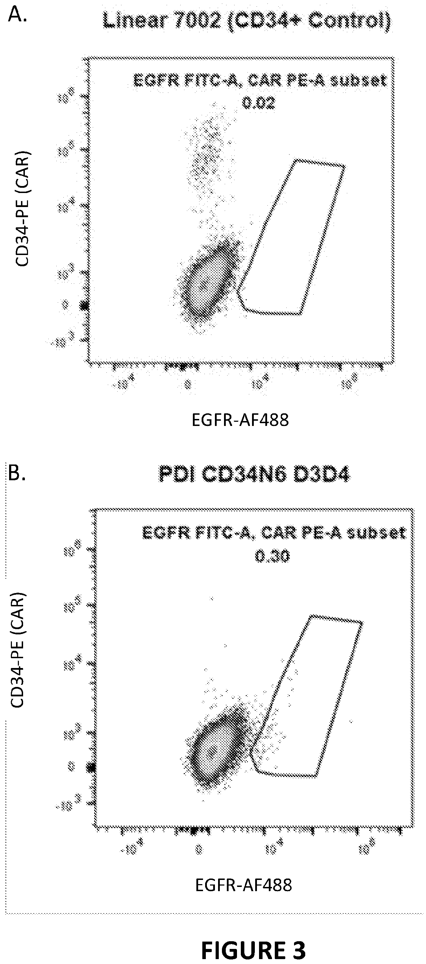

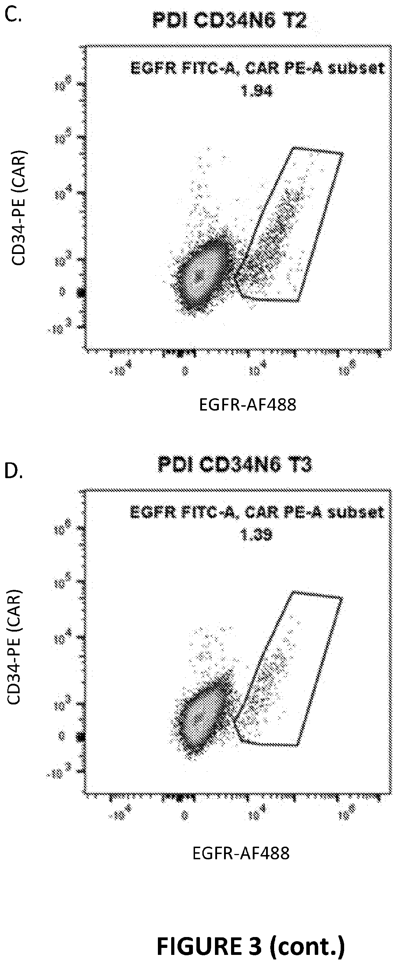

Figure 3 shows the frequencies and intensities of staining for human T cells expressing a chimeric antigen receptor (detected using CD34-PE) and a modified EGFR peptide (detected using cetuximab). Figure 3A represents a positive control for targeted insertion of the coding sequences into the T cell receptor alpha constant region gene. This positive control expresses a CAR that incorporates a CD34 epitope for detection, but does not express a modified EGFR peptide. Figure 3B shows binding properties of a CAR and a reference EGFR peptide which includes the wild-type EGFR transmembrane domain, wild-type EGFR Domain IV, and wild-type EGFR Domain III (SEQ ID NO: 3). Figure 3C shows binding properties of a CAR and the T2 EGFR variant (SEQ ID NO: 15). Figure 3D shows binding properties of a CAR and the T3 EGFR variant (SEQ ID NO: 16).

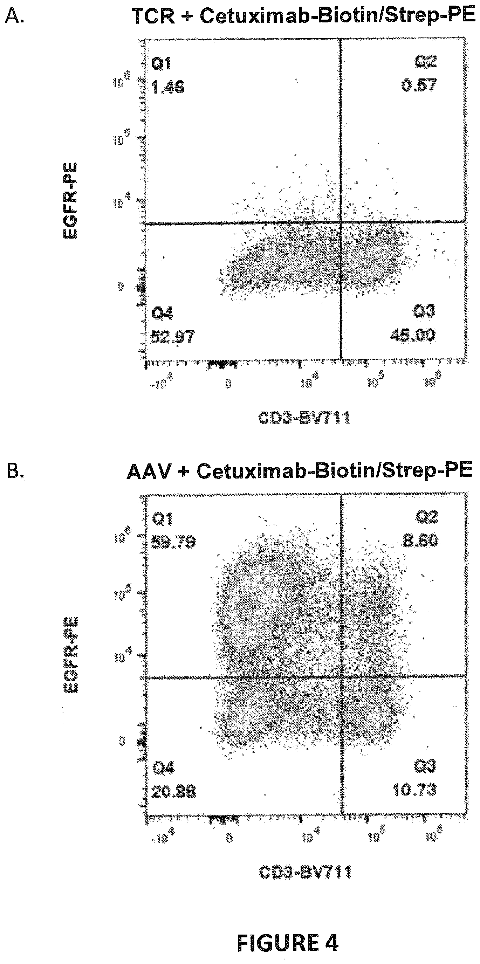

Figure 4 shows the expression level of the T2 EGFR variant (SEQ ID NO: 15), and cell-surface expression of the endogenous T cell receptor, in human T cells that have been transduced with an AAV to deliver a CAR/ T2 EGFR donor template, and nucleofected with a site-specific meganuclease to promote targeted insertion of the donor template into exon 1 of the T cell receptor alpha constant region gene. Figure 4A shows CD3 and EGFR staining data for human T cells nucleofected with the TRC 1-2x.87EE meganuclease, but not transduced with AAV. Figure 4B shows CD3 and EGFR staining data for human T cells nucleofected with the TRC 1-2x.87EE meganuclease, and subsequently transduced with AAV comprising the CAR/ T2 EGFR donor template.

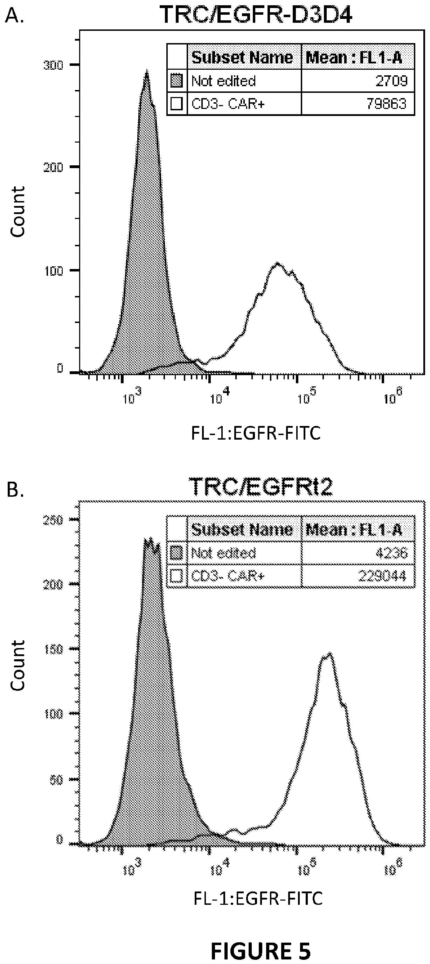

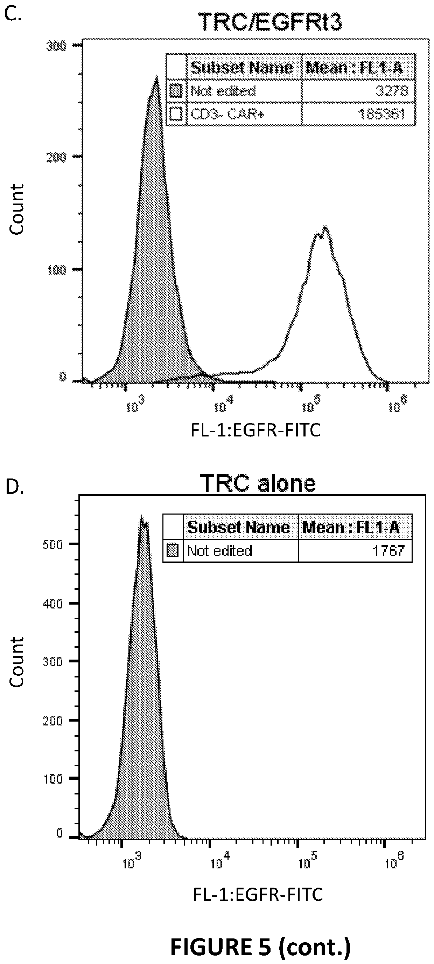

Figure 5 shows histograms illustrating the EGFR signal on non-edited cell populations (closed/shaded histograms) and the EGFR signal on corresponding CD3-/CAR+ cell populations (open/unshaded histograms) from the same sample. Figure 5A shows histograms from cells expressing the EGFR-D3D4 peptide (SEQ ID NO: 3). Figure 5B shows histograms from cells expressing the EGFRt2 peptide (SEQ ID NO: 15). Figure 5C shows histograms from cells expressing the EGFRt3 peptide (SEQ ID NO: 16). Figure 5D shows histograms from cells that were not transduced with AAV to express an EGFR peptide.

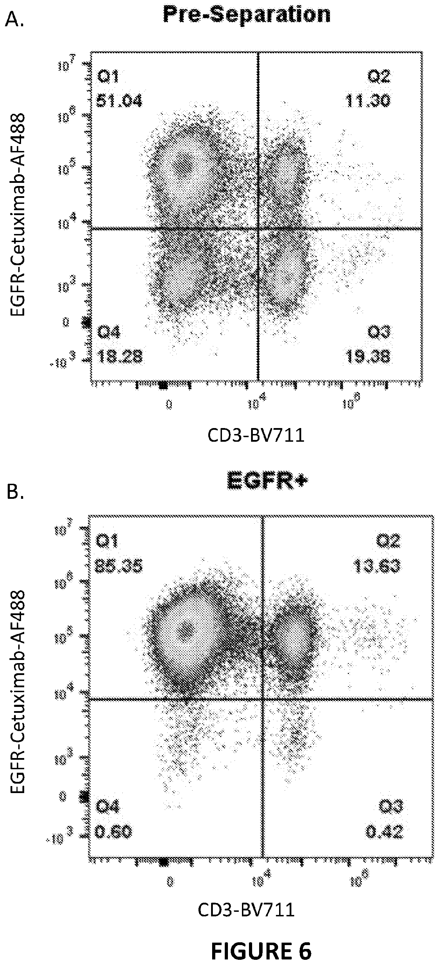

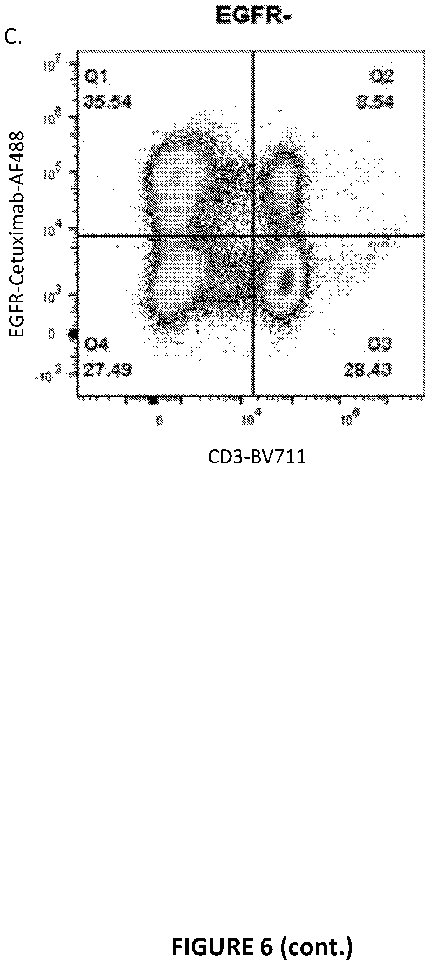

Figure 6 shows that labeling the T2 EGFR variant with biotinylated cetuximab can support magnetic enrichment of EGFR+ cells. Figure 6A illustrates staining of EGFR+ and EGFR- cells (Y axis), as well as TCR+ and TCR- cells (X axis), following nucleofection with the TRC 1-2x.87EE meganuclease and transduction with an AAV comprising the CAR/T2 EGFR (SEQ ID NO: 15) donor template. Figure 6B illustrates staining of EGFR+ cells separated from the sample with biotinylated cetuximab. Figure 6C illustrates staining of the EGFR- cell population.

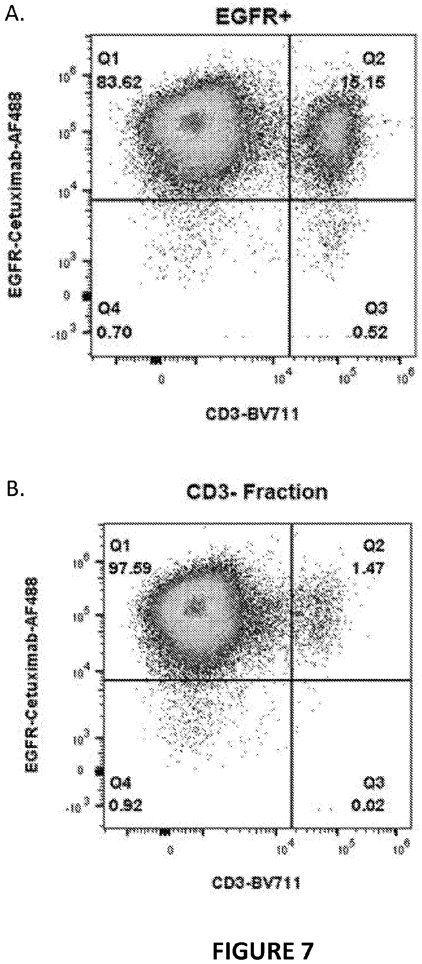

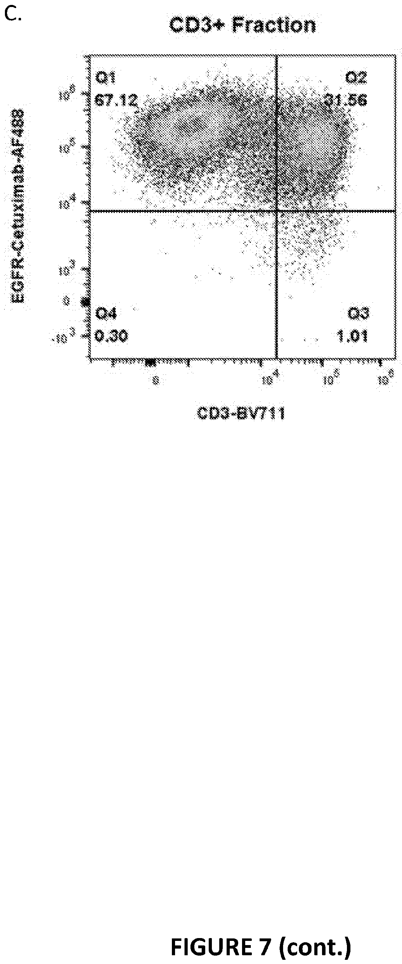

Figure 7 shows the results of depleting the CD3+ cells out of an EGFRenriched population. Figure 7A illustrates staining of an EGFR+ enriched population of cells, which express the T2 EGFR variant (SEQ ID NO: 15). Figure 7B illustrates staining of cells following CD3 depletion. Figure 7C illustrates staining of cells in the fraction retained from the CD3 depletion.

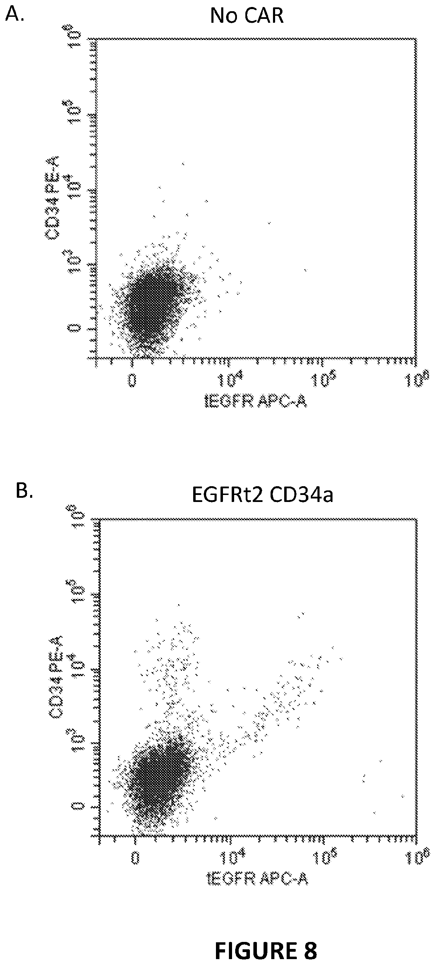

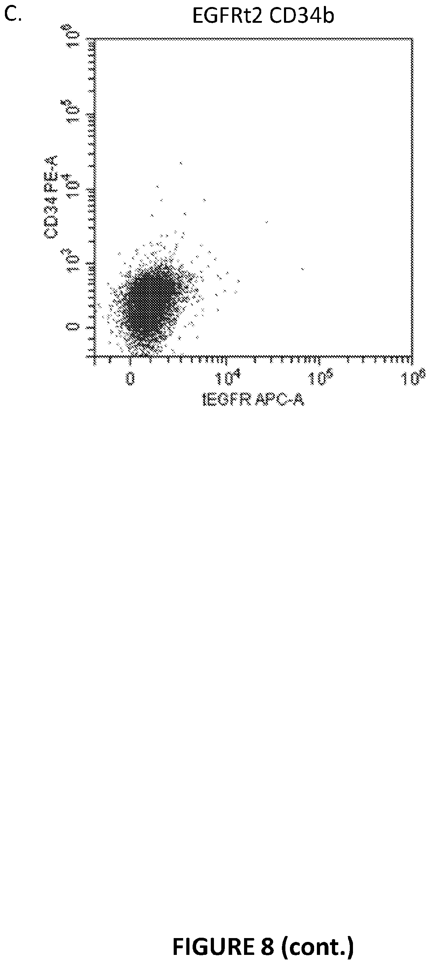

Figure 8 shows staining data of T cells expressing a chimeric antigen receptor and a T2 EGFR (SEQ ID NO: 15) that further incorporates a QBend10 CD34 epitope (SEQ ID NO: 28). Figure 8A shows staining data for a control T cell population nucleofected with mRNA for the TRC 1-2x.87EE meganuclease, but no CAR/T2 EGFR/QBend10 donor template. Figure 8B shows staining data for a T cell population nucleofected with mRNA for the TRC 1-2x.87EE meganuclease and a linearized CAR/T2 EGFR/QBend10 donor template, where the QBend10 epitope is positioned at the N-terminus of the EGFR Domain III in the coding sequence. Figure 8C shows staining data for a T cell population nucleofected with mRNA for the TRC 1-2x.87EE meganuclease and a linearized CAR/T2 EGFR/QBend10 donor template, wherein an additional arginine residue was positioned 5' upstream of the QBend10 epitope and a glycine residue was positioned between the QBend10 epitope and the N-terminus of the EGFR Domain III in the coding sequence.

### BRIEF DESCRIPTION OF THE SEQUENCES

SEQ ID NO: 1 sets forth the nucleic acid sequence encoding human EGFR isoform A.
SEQ ID NO: 2 sets forth the amino acid sequence of human EGFR isoform A precursor.
SEQ ID NO: 3 sets forth the amino acid sequence of a modified human EGFR comprising EGFR Domain III, EGFR Domain IV, and the EGFR transmembrane domain.
SEQ ID NO: 4 sets forth the nucleic acid sequence encoding a modified human EGFR comprising EGFR Domain III, EGFR Domain IV, and the EGFR transmembrane domain.
SEQ ID NO: 5 sets forth the amino acid sequence of EGFR Domain IV mutant 2.
SEQ ID NO: 6 sets forth the amino acid sequence of EGFR Domain IV mutant 3.
SEQ ID NO: 7 sets forth the amino acid sequence of EGFR Domain IV mutant 4.
SEQ ID NO: 8 sets forth the amino acid sequence of EGFR Domain IV mutant 5.
SEQ ID NO: 9 sets forth the amino acid sequence of EGFR Domain IV mutant 6.
SEQ ID NO: 10 sets forth the nucleic acid sequence encoding the EGFR Domain IV mutant 2.
SEQ ID NO: 11 sets forth the nucleic acid sequence encoding the EGFR Domain IV mutant 3.
SEQ ID NO: 12 sets forth the nucleic acid sequence encoding the EGFR Domain IV mutant 4.
SEQ ID NO: 13 sets forth the nucleic acid sequence encoding the EGFR Domain IV mutant 5.
SEQ ID NO: 14 sets forth the nucleic acid sequence encoding the EGFR Domain IV mutant 6.
SEQ ID NO: 15 sets forth the amino acid sequence of a modified human EGFR comprising EGFR Domain III, EGFR Domain IV mutant 2, and the EGFR transmembrane domain.
SEQ ID NO: 16 sets forth the amino acid sequence of a modified human EGFR comprising EGFR Domain III, EGFR Domain IV mutant 3, and the EGFR transmembrane domain.
SEQ ID NO: 17 sets forth the amino acid sequence of a modified human EGFR comprising EGFR Domain III, EGFR Domain IV mutant 4, and the EGFR transmembrane domain.
SEQ ID NO: 18 sets forth the amino acid sequence of a modified human EGFR comprising EGFR Domain III, EGFR Domain IV mutant 5, and the EGFR transmembrane domain.
SEQ ID NO: 19 sets forth the amino acid sequence of a modified human EGFR comprising EGFR Domain III, EGFR Domain IV mutant 6, and the EGFR transmembrane domain.
SEQ ID NO: 20 sets forth the nucleic acid sequence encoding a modified human EGFR comprising EGFR Domain III, EGFR Domain IV mutant 2, and the EGFR transmembrane domain.
SEQ ID NO: 21 sets forth the nucleic acid sequence encoding a modified human EGFR comprising EGFR Domain III, EGFR Domain IV mutant 3, and the EGFR transmembrane domain.
SEQ ID NO: 22 sets forth the nucleic acid sequence encoding a modified human EGFR comprising EGFR Domain III, EGFR Domain IV mutant 4, and the EGFR transmembrane domain.
SEQ ID NO: 23 sets forth the nucleic acid sequence encoding a modified human EGFR comprising EGFR Domain III, EGFR Domain IV mutant 5, and the EGFR transmembrane domain.
SEQ ID NO: 24 sets forth the nucleic acid sequence encoding a modified human EGFR comprising EGFR Domain III, EGFR Domain IV mutant 6, and the EGFR transmembrane domain.
SEQ ID NO: 25 sets forth the amino acid sequence of a modified human EGFR comprising EGFR Domain III and the EGFR transmembrane domain.
SEQ ID NO: 26 sets forth the nucleic acid sequence encoding a modified human EGFR comprising EGFR Domain III and the EGFR transmembrane domain.
SEQ ID NO: 27 sets forth the amino acid sequence of the GMCSFR alpha chain signal sequence.
SEQ ID NO: 28 sets forth the amino acid sequence of the QBend10 epitope.

### DETAILED DESCRIPTION OF THE INVENTION

### 1.1 References and Definitions

The patent and scientific literature referred to herein establishes knowledge that is available to those of skill in the art.

Unless otherwise defined, all technical and scientific terms used herein have the same meaning as commonly understood by one of ordinary skill in the art to which this disclosure belongs. The terminology used in the description of the present disclosure herein is for the purpose of describing particular embodiments only and is not intended to be limiting of the present disclosure.

As used herein, "a," "an," or "the" can mean one or more than one. For example, "a" cell can mean a single cell or a multiplicity of cells.

As used herein, unless specifically indicated otherwise, the word "or" is used in the inclusive sense of "and/or" and not the exclusive sense of "either/or."

As used herein, the terms "epidermal growth factor receptor" or "EGFR" are used interchangeably and refer to the human cell-surface peptide which binds to EGF. Generally, a human EGFR comprises four extracellular domains, referred to as Domain I, Domain II, Domain III, and Domain IV (from distal to proximal from the cell membrane). Human EGFR further comprises a transmembrane domain and cytoplasmic domains, including a juxtamembrane and a tyrosine kinase-binding domain. For example, the naturally-occurring EGFR referenced herein can include the human EGFR isoform A precursor set forth in NCBI Reference Sequence NP_005219.2, or other naturally-occurring isoforms or functional variants thereof. As used herein, EGFR Domain III refers to residues 1-171 of SEQ ID NO: 3, EGFR Domain IV refers to residues 172-311 of SEQ ID NO: 3, and the EGFR transmembrane domain refers to residues 312-335 of SEQ ID NO: 3.

As used herein, with respect to a protein, the term "engineered" or "recombinant" means having an altered amino acid sequence as a result of the application of genetic engineering techniques to nucleic acids which encode the protein, and cells or organisms which express the protein. With respect to a nucleic acid, the term "recombinant" means having an altered nucleic acid sequence as a result of the application of genetic engineering techniques. Genetic engineering techniques include, but are not limited to, PCR and DNA cloning technologies; transfection, transformation and other gene transfer technologies; homologous recombination; site-directed mutagenesis; and gene fusion. In accordance with this definition, a protein having an amino acid sequence identical to a naturally-occurring protein, but produced by cloning and expression in a heterologous host, is not considered recombinant.

As used herein, the term "wild-type" refers to the most common naturally occurring polynucleotide or polypeptide sequence responsible for a given phenotype. Whereas a wild-type allele or polypeptide can confer a normal phenotype in an organism, a mutant or variant allele or polypeptide can, in some instances, confer an altered phenotype.

As used herein with respect to recombinant proteins, the terms "modified" or "modification" means any insertion, deletion or substitution of an amino acid residue in the recombinant sequence relative to a reference sequence (*e.g.*, a wild-type or a native sequence). A modified EGFR Domain IV can mean a variant of the wild-type EGFR Domain IV which contains one or more changes, such as insertions, substitutions, or deletions of amino acids. Particularly, a modified EGFR Domain IV can mean a variant of the wild-type EGFR Domain IV that is truncated to remove approximately 30%, 35%, 40%, 45%, 50%, 55%, 60%, 65%, 70%, 75%, 80%, 85%, 90%, 95%, or more, of the wild-type amino acids.

The terms "recombinant DNA construct," "recombinant construct," "expression cassette," "expression construct," "chimeric construct," "construct," and "recombinant DNA fragment" are used interchangeably herein and are nucleic acid fragments. A recombinant construct comprises an artificial combination of nucleic acid fragments, including, without limitation, regulatory and coding sequences that are not found together in nature. For example, a recombinant DNA construct may comprise regulatory sequences and coding sequences that are derived from different sources, or regulatory sequences and coding sequences derived from the same source and arranged in a manner different than that found in nature. Such a construct may be used by itself or may be used in conjunction with a vector.

As used herein, a "vector" or "recombinant DNA vector" may be a construct that includes a replication system and sequences that are capable of transcription and translation of a polypeptide-encoding sequence in a given host cell. If a vector is used then the choice of vector is dependent upon the method that will be used to transform host cells as is well known to those skilled in the art. Vectors can include, without limitation, plasmid vectors and recombinant lentiviral or recombinant AAV vectors, or any other vector known in that art suitable for delivering a gene encoding a co-stimulatory domain of the present disclosure to a target cell. The skilled artisan is well aware of the genetic elements that must be present on the vector in order to successfully transform, select and propagate host cells comprising any of the isolated nucleotides or nucleic acid sequences of the present disclosure.

As used herein, a "vector" can also refer to a viral vector. Viral vectors can include, without limitation, retroviral vectors, lentiviral vectors, adenoviral vectors, and adeno-associated viral vectors (AAV).

As used herein, the term "operably linked" is intended to mean a functional linkage between two or more elements. For example, an operable linkage between a nucleic acid sequence encoding a nuclease as disclosed herein and a regulatory sequence (e.g., a promoter) is a functional link that allows for expression of the nucleic acid sequence encoding the nuclease. Operably linked elements may be contiguous or non-contiguous. When used to refer to the joining of two protein coding regions, by operably linked is intended that the coding regions are in the same reading frame.

As used herein, a "polycistronic" mRNA refers to a single messenger RNA that comprises two or more coding sequences (*i.e*., cistrons) and encodes more than one protein. A polycistronic mRNA can comprise any element known in the art to allow for the translation of two or more genes from the same mRNA molecule including, but not limited to, an IRES element or a 2A element (*e.g*., a T2A element, a P2A element, an E2A element, and an F2A element).

As used herein, "transfected" or "transformed" or "transduced" or "nucleofected" refers to a process by which exogenous nucleic acid is transferred or introduced into the host cell. A "transfected" or "transformed" or "transduced" cell is one which has been transfected, transformed or transduced with exogenous nucleic acid. The cell includes the primary subject cell and its progeny.

As used herein, a "human T cell" or "T cell" refers to a T cell isolated from a human donor. Human T cells, and cells derived therefrom, include isolated T cells that have not been passaged in culture, T cells that have been passaged and maintained under cell culture conditions without immortalization, and T cells that have been immortalized and can be maintained under cell culture conditions indefinitely.

As used herein, a "human natural killer cell" or "human NK cell" or "natural killer cell" or "NK cell" refers to a type of cytotoxic lymphocyte critical to the innate immune system. The role NK cells play is analogous to that of cytotoxic T-cells in the vertebrate adaptive immune response. NK cells provide rapid responses to virally infected cells and respond to tumor formation, acting at around 3 days after infection. Human NK cells, and cells derived therefrom, include isolated NK cells that have not been passaged in culture, NK cells that have been passaged and maintained under cell culture conditions without immortalization, and NK cells that have been immortalized and can be maintained under cell culture conditions indefinitely.

As used herein, a "control" or "control cell" refers to a cell that provides a reference point for measuring changes in genotype or phenotype of a genetically-modified cell. A control cell may comprise, for example: (a) a wild-type cell, *i.e*., of the same genotype as the starting material for the genetic alteration which resulted in the genetically-modified cell; (b) a cell of the same genotype as the genetically-modified cell but which has been transformed with a null construct (*i.e.*, with a construct which has no known effect on the trait of interest); or, (c) a cell genetically identical to the genetically-modified cell but which is not exposed to conditions, stimuli, or further genetic modifications that would induce expression of altered genotype or phenotype.

As used herein, a "chimeric antigen receptor" or "CAR" refers to an engineered receptor that grafts specificity for an antigen or other ligand or molecule onto an immune effector cell (*e.g.,* a T cell or NK cell). A chimeric antigen receptor typically comprises at least an extracellular ligand-binding domain or moiety and an intracellular domain that comprises one or more signaling domains and/or co-stimulatory domains.

In some embodiments, the extracellular ligand-binding domain or moiety is in the form of a single-chain variable fragment (scFv) derived from a monoclonal antibody, which provides specificity for a particular epitope or antigen (*e.g.,* an epitope or antigen preferentially present on the surface of a cell, such as a cancer cell or other disease-causing cell or particle). In some embodiments, the scFv is attached via a linker sequence. In some embodiments, the extracellular ligand-binding domain is specific for any antigen or epitope of interest. In some embodiments, the scFv is humanized or fully human. In some embodiments, the extracellular domain of a chimeric antigen receptor comprises an autoantigen (see, Payne et al. (2016) Science, Vol. 353 (6295): 179-184), which is recognized by autoantigen-specific B cell receptors on B lymphocytes, thus directing T cells to specifically target and kill autoreactive B lymphocytes in antibody-mediated autoimmune diseases. Such CARs can be referred to as chimeric autoantibody receptors (CAARs).

Intracellular signaling domains are cytoplasmic domains which transmit an activation signal to the cell following binding of the extracellular domain. An intracellular signaling domain of the presently disclosed CARs can be any intracellular signaling domain of interest that is known in the art. Such cytoplasmic signaling domains can include, without limitation, CD3 ζ.

In some embodiments, the intracellular domain also includes one or more intracellular co-stimulatory domains. As used herein, a "co-stimulatory domain" refers to a polypeptide domain which transmits an intracellular proliferative and/or cell-survival signal upon activation. Activation of a co-stimulatory domain may occur following homodimerization of two co-stimulatory domain polypeptides. Activation may also occur, for example, following activation of a construct comprising the co-stimulatory domain (*e.g*., a chimeric antigen receptor or an inducible regulatory construct). Generally, a co-stimulatory domain can be derived from a transmembrane co-stimulatory receptor, particularly from an intracellular portion of a co-stimulatory receptor. Non-limiting examples of co-stimulatory polypeptides include, but are not limited to, those co-stimulatory domains described herein (*i.e.,* Novel6 or N6), 4-1BB, CD28, ICOS, OX-40, CD27, and others known in the art.

As used herein, a "co-stimulatory signal" refers to an intracellular signal induced by a co-stimulatory domain that promotes cell proliferation, expansion of a cell population *in vitro* and/or *in vivo,* promotes cell survival, modulates (*e.g*., upregulates or downregulates) the secretion of cytokines, and/or modulates the production and/or secretion of other immunomodulatory molecules. In some embodiments, a co-stimulatory signal is induced following homodimerization of two co-stimulatory domain polypeptides. In some embodiments, a co-stimulatory signal is induced following activation of a construct comprising the co-stimulatory domain (*e.g*., a chimeric antigen receptor or an inducible regulatory construct).

As used herein, the term "activation" refers to the state of a cell (*e.g*., a T cell) that has been sufficiently stimulated to induce detectable effector function. In some embodiments, activation is associated with induced cytokine production and/or induced cell proliferation and expansion.

In some embodiments, a chimeric antigen receptor further includes additional structural elements, including a transmembrane domain which is attached to the extracellular ligand-binding domain via a hinge or junction sequence.

As used herein, an "exogenous T cell receptor" or "exogenous TCR" refers to a TCR whose sequence is introduced into the genome of an immune effector cell (*e.g.,* a human T cell) that may or may not endogenously express the TCR. Expression of an exogenous TCR on an immune effector cell can confer specificity for a specific epitope or antigen (*e.g*., an epitope or antigen preferentially present on the surface of a cancer cell or other disease-causing cell or particle). Such exogenous T cell receptors can comprise alpha and beta chains or, alternatively, may comprise gamma and delta chains. Exogenous TCRs useful in the invention may have specificity to any antigen or epitope of interest.

As used herein, the term "anti-tumor activity" or "anti-tumor effect" refers to a biological effect which can be manifested by a decrease in tumor volume, a decrease in the number of tumor cells, a decrease in the number of metastases, an increase in life expectancy, or amelioration of various physiological symptoms associated with the cancerous condition. An "anti-tumor effect" can also be manifested by the ability of the genetically-modified cells of the present disclosure in prevention of the occurrence of tumor in the first place.

As used herein, the terms "T cell receptor alpha gene" or "TCR alpha gene" are interchangeable and refer to the locus in a T cell which encodes the T cell receptor alpha subunit. The T cell receptor alpha can refer to NCBI gene ID number 6955, before or after rearrangement. Following rearrangement, the T cell receptor alpha gene comprises an endogenous promoter, rearranged V and J segments, the endogenous splice donor site, an intron, the endogenous splice acceptor site, and the T cell receptor alpha constant region locus, which comprises the subunit coding exons.

As used herein, the term "T cell receptor alpha constant region gene" refers to the coding sequence of the T cell receptor alpha gene. The T cell receptor alpha constant region gene includes the wild-type sequence, and functional variants thereof, identified by NCBI Gen ID NO. 28755.

As used herein, the phrases "intron within the T cell receptor alpha gene" and "the targeted 5' intron" refer to the intron which in the rearranged T cell receptor alpha gene is positioned 5' upstream of exon 1 of the T cell receptor alpha constant region gene, 3' downstream of the V and J segments, and is flanked by the endogenous splice donor site and the endogenous splice acceptor site.

As used herein, the term "endogenous splice donor site" refers to the naturally-occurring splice donor site positioned 3' downstream of the endogenous TCR alpha gene promoter and the rearranged V and J segments, and 5' upstream of the targeted intron. Likewise, the "endogenous splice acceptor site" refers to the naturally-occurring splice acceptor site that is 3' downstream of the targeted intron and immediately 5' upstream of exon 1 of the T cell receptor alpha constant region gene. Endogenous splice donor sites and endogenous splice acceptor sites can be identified in a gene by methods known in the art, such as those described by Desmet et al. (Nucleic Acid Research (2009) 37(9): e67). The term "functional" as it relates to the endogenous splice donor site and the endogenous splice acceptor site refers to their ability to pair in order to execute splicing of the intervening intron sequence.

As used herein, the term "exogenous splice acceptor site" refers to a splice acceptor site which is comprised by the exogenous sequence of interest and is introduced into the targeted 5' intron. The exogenous splice acceptor site can comprise a sequence naturally present in the human T cell receptor alpha gene, or can comprise a splice acceptor sequence (e.g., a consensus or heterologous sequence) which is not naturally present in the gene. The exogenous splice acceptor site may further comprise an exogenous branch site if necessary to promote splicing of the intron. Such a branch site may comprise a sequence which is naturally present in the T cell receptor alpha gene, or can comprise a branch site sequence (*e.g.,* a consensus or heterologous sequence) which is not naturally present in the gene.

As used herein, the term "homologous recombination" or "HR" refers to the natural, cellular process in which a double-stranded DNA-break is repaired using a homologous DNA sequence as the repair template (see, *e.g.,* Cahill et al. (2006), Front. Biosci. 11:1958-1976). The homologous DNA sequence may be an endogenous chromosomal sequence or an exogenous nucleic acid that was delivered to the cell.

As used herein, the term "non-homologous end-joining" or "NHEJ" refers to the natural, cellular process in which a double-stranded DNA-break is repaired by the direct joining of two non-homologous DNA segments (see, *e.g.,* Cahill et al. (2006), Front. Biosci. 11:1958-1976). DNA repair by non-homologous end-joining is error-prone and frequently results in the untemplated addition or deletion of DNA sequences at the site of repair. In some instances, cleavage at a target recognition sequence results in NHEJ at a target recognition site. Nuclease-induced cleavage of a target site in the coding sequence of a gene followed by DNA repair by NHEJ can introduce mutations into the coding sequence, such as frameshift mutations, that disrupt gene function. Thus, engineered nucleases can be used to effectively knock-out a gene in a population of cells.

As used herein, the term "reduced" refers to any reduction in the symptoms or severity of a disease or any reduction in the proliferation or number of cancerous cells. In either case, such a reduction may be up to 5%, 10%, 20%, 30%, 40%, 50%, 60%, 70%, 80%, 90%, 95%, or up to 100%. Accordingly, the term "reduced" encompasses both a partial reduction and a complete reduction of a disease state.

As used herein, the term "increased" refers to any increase in the activation, proliferation, or cytokine signaling of a cell genetically-modified to comprise a co-stimulatory domain disclosed herein, or an active fragment or variant thereof. Such an increase may be up to 5%, 10%, 20%, 30%, 40%, 50%, 60%, 70%, 80%, 90%, 95%, or up to 100%, or more. Any method can be used to measure an increase in the activation, proliferation, or cytokine signaling of a cell. For example, increased activation and/or cytokine expression can encompass an increase of expression of any one of IFN-γ, IL-2, TNF-α, or any other cytokine that could be used to determine a change in cell activation and/or proliferation. In some embodiments, an increase in proliferation encompasses an increase in cell number or cell division, and includes an expansion of a cell population.

The term "effective amount" or "therapeutically effective amount" refers to an amount sufficient to effect beneficial or desirable biological and/or clinical results. The therapeutically effective amount will vary depending on the therapeutic (*e.g*., genetically-modified cell, CAR-T cell, CAR-NK cell) formulation or composition, the disease and its severity, and the age, weight, physical condition and responsiveness of the subject to be treated. In specific embodiments, an effective amount of a cell comprising a modified EGFR peptide disclosed herein or pharmaceutical compositions disclosed herein reduces at least one symptom or the progression of a disease.

As used herein, the term "treat" or "treatment" means to reduce the frequency or severity of at least one sign or symptom of a disease or disorder experienced by a subject.

As used herein, the term "cancer" should be understood to encompass any neoplastic disease (whether invasive or metastatic) which is characterized by abnormal and uncontrolled cell division causing malignant growth or tumor.

As used herein, the term "carcinoma" refers to a malignant growth made up of epithelial cells.

As used herein, the term "leukemia" refers to malignancies of the hematopoietic organs/systems and is generally characterized by an abnormal proliferation and development of leukocytes and their precursors in the blood and bone marrow.

As used herein, the term "sarcoma" refers to a tumor which is made up of a substance like the embryonic connective tissue and is generally composed of closely packed cells embedded in a fibrillary, heterogeneous, or homogeneous substance.

As used herein, the term "melanoma" refers to a tumor arising from the melanocytic system of the skin and other organs.

As used herein, the term "lymphoma" refers to a group of blood cell tumors that develop from lymphocytes.

As used herein, the term "blastoma" refers to a type of cancer that is caused by malignancies in precursor cells or blasts (immature or embryonic tissue).

As used herein, the term "meganuclease" refers to an endonuclease that binds double-stranded DNA at a recognition sequence that is greater than 12 base pairs. In some embodiments, the recognition sequence for a meganuclease of the present disclosure is 22 base pairs. A meganuclease can be an endonuclease that is derived from I-CreI, and can refer to an engineered variant of I-CreI that has been modified relative to natural I-CreI with respect to, for example, DNA-binding specificity, DNA cleavage activity, DNA-binding affinity, or dimerization properties. Methods for producing such modified variants of I-CreI are known in the art (*e.g.* WO 2007/047859). A meganuclease as used herein binds to double-stranded DNA as a heterodimer. A meganuclease may also be a "single-chain meganuclease" in which a pair of DNA-binding domains are joined into a single polypeptide using a peptide linker. The term "homing endonuclease" is synonymous with the term "meganuclease." Meganucleases of the present disclosure are substantially non-toxic when expressed in cells, particularly in human T cells, such that cells can be transfected and maintained at 37°C without observing deleterious effects on cell viability or significant reductions in meganuclease cleavage activity when measured using the methods described herein.

As used herein, the term "single-chain meganuclease" refers to a polypeptide comprising a pair of nuclease subunits joined by a linker. A single-chain meganuclease has the organization: N-terminal subunit - Linker - C-terminal subunit. The two meganuclease subunits will generally be non-identical in amino acid sequence and will recognize non-identical DNA sequences. Thus, single-chain meganucleases typically cleave pseudopalindromic or non-palindromic recognition sequences. A single-chain meganuclease may be referred to as a "single-chain heterodimer" or "single-chain heterodimeric meganuclease" although it is not, in fact, dimeric. For clarity, unless otherwise specified, the term "meganuclease" can refer to a dimeric or single-chain meganuclease.

As used herein, the term "linker" refers to an exogenous peptide sequence used to join two meganuclease subunits into a single polypeptide. A linker may have a sequence that is found in natural proteins, or may be an artificial sequence that is not found in any natural protein. A linker may be flexible and lacking in secondary structure or may have a propensity to form a specific three-dimensional structure under physiological conditions. A linker can include, without limitation, any of those encompassed by U.S. Patent Nos. 8,445,251 and 9,434,931.

As used herein, the term "zinc finger nuclease" or "ZFN" refers to chimeric proteins comprising a zinc finger DNA-binding domain fused to a nuclease domain from an endonuclease or exonuclease, including but not limited to a restriction endonuclease, homing endonuclease, S1 nuclease, mung bean nuclease, pancreatic DNAse I, micrococcal nuclease, and yeast HO endonuclease. Nuclease domains useful for the design of zinc finger nuclease include those from a Type IIs restriction endonuclease, including but not limited to FokI, FoM, StsI restriction enzyme. Additional Type IIs restriction endonucleases are described in International Publication No. WO 2007/014275. The structure of a zinc finger domain is stabilized through coordination of a zinc ion. DNA binding proteins comprising one or more zinc finger domains bind DNA in a sequence-specific manner. The zinc finger domain can be a native sequence or can be redesigned through rational or experimental means to produce a protein which binds to a pre-determined DNA sequence ~18 basepairs in length. See, for example, U.S. Pat. Nos. 5,789,538, 5,925,523, 6,007,988, 6,013,453, 6,200,759, and International Publication Nos. WO 95/19431, WO 96/06166, WO 98/53057, WO 98/54311, WO 00/27878, WO 01/60970, WO 01/88197, and WO 02/099084. By fusing this engineered protein domain to a nuclease domain, such as FokI nuclease, it is possible to target DNA breaks with genome-level specificity. The selection of target sites, zinc finger proteins and methods for design and construction of zinc finger nucleases are known to those of skill in the art and are described in detail in U.S. Publications Nos. 20030232410, 20050208489, 2005064474, 20050026157, 20060188987 and International Publication No. WO 07/014275.

As used herein, the term "TALEN" refers to an endonuclease comprising a DNA-binding domain comprising a plurality of TAL domain repeats fused to a nuclease domain or an active portion thereof from an endonuclease or exonuclease, including but not limited to a restriction endonuclease, homing endonuclease, S1 nuclease, mung bean nuclease, pancreatic DNAse I, micrococcal nuclease, and yeast HO endonuclease. *See,* for example, Christian et al. (2010) Genetics 186:757-761. Nuclease domains useful for the design of TALENs include those from a Type IIs restriction endonuclease, including but not limited to FokI, FoM, StsI, HhaI, HindIII, Nod, BbvCI, EcoRI, BglI, and AlwI. Additional Type IIs restriction endonucleases are described in International Publication No. WO 2007/014275. In some embodiments, the nuclease domain of the TALEN is a FokI nuclease domain or an active portion thereof. TAL domain repeats can be derived from the TALE (transcription activator-like effector) family of proteins used in the infection process by plant pathogens of the *Xanthomonas* genus. TAL domain repeats are 33-34 amino acid sequences with divergent 12^{th} and 13^{th} amino acids. These two positions, referred to as the repeat variable dipeptide (RVD), are highly variable and show a strong correlation with specific nucleotide recognition. Each base pair in the DNA target sequence is contacted by a single TAL repeat, with the specificity resulting from the RVD. In some embodiments, the TALEN comprises 16-22 TAL domain repeats. DNA cleavage by a TALEN requires two DNA recognition regions flanking a nonspecific central region (*i.e.,* the "spacer"). The term "spacer" in reference to a TALEN refers to the nucleic acid sequence that separates the two nucleic acid sequences recognized and bound by each monomer constituting a TALEN. The TAL domain repeats can be native sequences from a naturally-occurring TALE protein or can be redesigned through rational or experimental means to produce a protein which binds to a pre-determined DNA sequence (see, for example, Boch et al. (2009) Science 326(5959):1509-1512 and Moscou and Bogdanove (2009) Science 326(5959):1501). See also, U.S. Publication No. 20110145940 and International Publication No. WO 2010/079430 for methods for engineering a TALEN to recognize a specific sequence and examples of RVDs and their corresponding target nucleotides. In some embodiments, each nuclease (*e.g*., FokI) monomer can be fused to a TAL effector sequence that recognizes a different DNA sequence, and only when the two recognition sites are in close proximity do the inactive monomers come together to create a functional enzyme.

As used herein, the term "compact TALEN" refers to an endonuclease comprising a DNA-binding domain with one or more TAL domain repeats fused in any orientation to any portion of the I-TevI homing endonuclease or any of the endonucleases listed in Table 2 in U.S. Application No. 20130117869, including but not limited to MmeI, EndA, End1, I-BasI, I-TevII, I-TevIII, I-TwoI, MspI, MvaI, NucA, and NucM. Compact TALENs do not require dimerization for DNA processing activity, alleviating the need for dual target sites with intervening DNA spacers. In some embodiments, the compact TALEN comprises 16-22 TAL domain repeats.

As used herein, the term "CRISPR" refers to a caspase-based endonuclease comprising a caspase, such as Cas9, and a guide RNA that directs DNA cleavage of the caspase by hybridizing to a recognition site in the genomic DNA. The caspase component of a CRISPR is an RNA-guided DNA endonuclease. In certain embodiments, the caspase is a class II Cas enzyme. In some of these embodiments, the caspase is a class II, type II enzyme, such as Cas9. In other embodiments, the caspase is a class II, type V enzyme, such as Cpf1. The guide RNA comprises a direct repeat and a guide sequence (often referred to as a spacer in the context of an endogenous CRISPR system), which is complementary to the target recognition site. In certain embodiments, the CRISPR further comprises a tracrRNA (transactivating CRISPR RNA) that is complementary (fully or partially) to a direct repeat sequence (sometimes referred to as a tracr-mate sequence) present on the guide RNA. In particular embodiments, the caspase can be mutated with respect to a corresponding wild-type enzyme such that the enzyme lacks the ability to cleave one strand of a target polynucleotide, functioning as a nickase, cleaving only a single strand of the target DNA. Non-limiting examples of caspase enzymes that function as a nickase include Cas9 enzymes with a D10A mutation within the RuvC I catalytic domain, or with a H840A, N854A, or N863A mutation.

As used herein, the term "megaTAL" refers to a single-chain nuclease comprising a transcription activator-like effector (TALE) DNA binding domain with an engineered, sequence-specific homing endonuclease.

As used herein, the term "recognition sequence" refers to a DNA sequence that is bound and cleaved by an endonuclease. In the case of a meganuclease, a recognition sequence comprises a pair of inverted, 9 base pair "half sites" which are separated by four basepairs. In the case of a single-chain meganuclease, the N-terminal domain of the protein contacts a first half-site and the C-terminal domain of the protein contacts a second half-site. Cleavage by a meganuclease produces four base pair 3' "overhangs". "Overhangs," or "sticky ends" are short, single-stranded DNA segments that can be produced by endonuclease cleavage of a double-stranded DNA sequence. In the case of meganucleases and single-chain meganucleases derived from I-CreI, the overhang comprises bases 10-13 of the 22 base pair recognition sequence. In the case of a compact TALEN, the recognition sequence can comprises a first CNNNGN sequence that is recognized by the I-TevI domain, followed by a non-specific spacer 4-16 basepairs in length, followed by a second sequence 16-22 bp in length that is recognized by the TAL-effector domain (this sequence typically has a 5' T base). Cleavage by a Compact TALEN produces two base pair 3' overhangs. In the case of a CRISPR, the recognition sequence is the sequence, typically 16-24 basepairs, to which the guide RNA binds to direct Cas9 cleavage. Full complementarity between the guide sequence and the recognition sequence is not necessarily required to effect cleavage. Cleavage by a CRISPR can produce blunt ends (such as by a class II, type II caspase) or overhanging ends (such as by a class II, type V caspase), depending on the caspase. In those embodiments wherein a CpfI caspase is utilized, cleavage by the CRISPR complex comprising the same will result in 5' overhangs and in certain embodiments, 5 nucleotide 5' overhangs. Each caspase enzyme also requires the recognition of a PAM (protospacer adjacent motif) sequence that is near the recognition sequence complementary to the guide RNA. The precise sequence, length requirements for the PAM, and distance from the target sequence differ depending on the caspase enzyme, but PAMs are typically 2-5 base pair sequences adjacent to the target/recognition sequence. PAM sequences for particular caspase enzymes are known in the art (see, for example, U.S. Patent No. 8,697,359 and U.S. Publication No. 20160208243) and PAM sequences for novel or engineered caspase enzymes can be identified using methods known in the art, such as a PAM depletion assay (see, for example, Karvelis et al. (2017) Methods 121-122:3-8,

As used herein, the term "target site" or "target sequence" refers to a region of the chromosomal DNA of a cell comprising a recognition sequence for a nuclease.

As used herein with respect to both amino acid sequences and nucleic acid sequences, the terms "percent identity," "sequence identity," "percentage similarity," "sequence similarity," and the like, refer to a measure of the degree of similarity of two sequences based upon an alignment of the sequences which maximizes similarity between aligned amino acid residues or nucleotides, and which is a function of the number of identical or similar residues or nucleotides, the number of total residues or nucleotides, and the presence and length of gaps in the sequence alignment. A variety of algorithms and computer programs are available for determining sequence similarity using standard parameters. As used herein, sequence similarity is measured using the BLASTp program for amino acid sequences and the BLASTn program for nucleic acid sequences, both of which are available through the National Center for Biotechnology Information (www.ncbi.nlm.nih.gov), and are described in, for example, Altschul et al. (1990), J. Mol. Biol. 215:403-410; Gish and States (1993), Nature Genet. 3:266-272; Madden et al. (1996), Meth. Enzymol.266:131-141*;* Altschul et al. (1997), Nucleic Acids Res. 25:33 89-3402); Zhang et al. (2000), J. Comput. Biol. 7(1-2):203-14. As used herein, percent similarity of two amino acid sequences is the score based upon the following parameters for the BLASTp algorithm: word size=3; gap opening penalty=-11; gap extension penalty=-1; and scoring matrix=BLOSUM62. As used herein, percent similarity of two nucleic acid sequences is the score based upon the following parameters for the BLASTn algorithm: word size=11; gap opening penalty=-5; gap extension penalty=-2; match reward=1; and mismatch penalty=-3.

As used herein with respect to modifications of two proteins or amino acid sequences, the term "corresponding to" is used to indicate that a specified modification in the first protein is a substitution of the same amino acid residue as in the modification in the second protein, and that the amino acid position of the modification in the first proteins corresponds to or aligns with the amino acid position of the modification in the second protein when the two proteins are subjected to standard sequence alignments (*e.g*., using the BLASTp program). Thus, the modification of residue "X" to amino acid "A" in the first protein will correspond to the modification of residue "Y" to amino acid "A" in the second protein if residues X and Y correspond to each other in a sequence alignment, and despite the fact that X and Y may be different numbers.

As used herein, the recitation of a numerical range for a variable is intended to convey that the present disclosure may be practiced with the variable equal to any of the values within that range. Thus, for a variable which is inherently discrete, the variable can be equal to any integer value within the numerical range, including the end-points of the range. Similarly, for a variable which is inherently continuous, the variable can be equal to any real value within the numerical range, including the end-points of the range. As an example, and without limitation, a variable which is described as having values between 0 and 2 can take the values 0, 1 or 2 if the variable is inherently discrete, and can take the values 0.0, 0.1, 0.01, 0.001, or any other real values ≧0 and ≦2 if the variable is inherently continuous.

### 2.1 Principle of the Invention

The present disclosure is based, in part, on the discovery that truncated EGFR peptides modified to mutate Domain IV are smaller in size, aiding packaging and delivery of nucleic acid molecules encoding the same, and unexpectedly exhibit superior binding to anti-EGFR antibodies, when compared to previously described truncated EGFR peptides.

The epidermal growth factor receptor (EGFR), also known as ErbB1 and HER1, is a transmembrane cell-surface receptor for members of the epidermal growth factor family of extracellular ligands. Upon activation by its ligands, EGFR homodimerizes or heterodimerizes with family member ErbB2/Her2/neu, which stimulates its intracellular protein tyrosine kinase activity and autophosphorylation of several tyrosine residues in the carboxyl-terminal domain of EGFR. Autophosphorylation elicits downstream activation and cellular signaling processes that result in DNA synthesis and cellular proliferation, in addition to other cellular functions including cellular differentiation, motility, and survival (Wang et al. (2004) J Biol Chem 279:53848-53856). The modified EGFR peptides disclosed herein lack the ability to stimulate cellular signaling due to the absence of the intracellular tyrosine kinase domain, but rather find use in enriching genetically-modified cells expressing the modified EGFR peptides, and for the *in vivo* depletion of cells expressing the modified EGFR peptides.

There are nine known EGFR isoforms in humans. *See* NCBI Gene ID number 1956. The canonical human EGFR sequence is that of isoform A, the longest isoform with 1210 amino acids (including the 24 amino acid amino-terminal signal sequence that is cleaved to produce the mature protein of 1186 amino acids). The amino acid sequence for human EGFR isoform A precursor is set forth in SEQ ID NO: 2 and NCBI Accession No. NP_005219.2, which is encoded by transcript variant 1 set forth in SEQ ID NO: 1 and NCBI Accession No. NM_005228.4. The amino acid sequences of EGFR isoforms B, C, D, E, F, G, H, and I are set forth in NCBI Accession Nos.: NP_958439.1, NP_958440.1, NP_958441.1, NP_001333826.1, NP_001333827.1, NP_001333828.1, NP_001333829.1, and NP_001333870.1, respectively, and are encoded by the nucleotide sequences set forth in NCBI Accession Nos: NM_201282.1, NM_201283.1, NM_201284.1, NM_001346897.1, NM_001346898.1, NM_001346899.1, NM_001346900.1, and NM _001346941.1, respectively.

The overall domain structure of EGFR is known in the art (see, for example, Ferguson (2008) Annual Review of Biophysics 37:353-373) and is described herein based on the canonical human EGFR isoform A precursor sequence. The human EGFR precursor comprises a 24 amino acid signal sequence (amino acid residues 1-24 of SEQ ID NO: 2) that is not present on the mature protein. The extracellular portion of the EGFR protein includes an EGFR Domain I, also known as L1 (amino acid residues 25-189 of SEQ ID NO: 2); an EGFR Domain II, also known as S1 (amino acid residues 190-333 of SEQ ID NO: 2); an EGFR Domain III, also known as L2 (amino acid residues 334-504 of SEQ ID NO: 2); and an EGFR Domain IV, also known as S2 (amino acid residues 505-644 of SEQ ID NO: 2). The transmembrane domain corresponds to amino acid residues 645-668 of SEQ ID NO: 2. The cytoplasmic portion of EGFR includes a juxtamembrane domain (amino acid residues 669-709 of SEQ ID NO: 2); a tyrosine kinase domain (amino acid residues 710-977 of SEQ ID NO: 2); and a cytoplasmic regulatory region (amino acid residues 978 to 1210 of SEQ ID NO: 2).

A "modified EGFR" refers to an EGFR sequence that is not found in nature. The presently disclosed modified EGFR peptides comprise an EGFR Domain III, a modified EGFR Domain IV, wherein said modified EGFR Domain IV is a truncated EGFR Domain IV, and an EGFR transmembrane domain, but do not comprise an EGFR Domain I, an EGFR Domain II, an EGFR juxtamembrane domain, or an EGFR tyrosine kinase domain. In certain embodiments, the modified EGFR peptide completely lacks all cytoplasmic sequences that span the juxtamembrane domain, the tyrosine kinase domain and the cytoplasmic regulatory domain and corresponds to amino acid residues 669-1210 of SEQ ID NO: 2.

The modified EGFR peptides comprise a modified EGFR Domain IV. As used herein, a "modified EGFR Domain IV" refers to an EGFR Domain IV comprising a sequence that is not found in nature. By comparison to the wild-type Domain IV sequence, the modified Domain IV sequences encompassed by the invention have been truncated. For example, in specific embodiments, modified Domain IV sequences encompass sequences that have been truncated at positions following each of the disulfide bonds in the wild-type Domain IV sequence. In some embodiments, the modified EGFR Domain IV comprises the sequence set forth in SEQ ID NOs: 5-9. In certain embodiments, the presently disclosed modified EGFR peptides comprise a modified EGFR Domain IV that has at least about 40%, about 45%, about 50%, about 55%, about 60%, about 65%, about 70%, about 75%, about 80%, about 85%, about 90%, about 91%, about 92%, about 93%, about 94%, about 95%, about 96%, about 97%, about 98%, or about 99%, sequence identity to any one of the sequences set forth in SEQ ID NOs: 5-9. In other embodiments, the presently disclosed modified EGFR peptides lack an EGFR Domain IV.

The presently disclosed modified EGFR peptides can bind to an anti-EGFR antibody. Such an anti-EGFR antibody binds to the extracellular domain of EGFR so as to be useful in enriching cells that express the modified EGFR peptides on the cellular surface and in some embodiments, inducing complement-dependent cytotoxicity (CDC) and/or antibody-dependent cell-mediated cytotoxicity (ADCC) to deplete genetically-modified cells expressing the modified EGFR peptides that have been administered to a subject. Thus, in some embodiments, the anti-EGFR antibody that binds to the modified EGFR peptide is capable of inducing ADCC, CDC, or both. In some embodiments, the anti-EGFR antibody that binds to the modified EGFR peptides competes with cetuximab for binding to EGFR or binds to the same or similar epitope as cetuximab. In particular embodiments, the anti-EGFR antibody that binds to the modified EGFR peptides is cetuximab or comprises the six complementarity determining regions (CDRs) of cetuximab. In certain embodiments, the modified EGFR peptides bind to an anti-EGFR antibody via EGFR Domain III. Non-limiting examples of anti-EGFR antibodies that bind to the modified EGFR peptides disclosed herein include cetuximab, panitumumab, matuzumab, zalutumumab, and nimotuzumab.

In certain embodiments, the presently disclosed modified EGFR peptides exhibit enhanced binding by an anti-EGFR antibody when compared to the EGFRt peptide described by the `894 publication and set forth herein as SEQ ID NO: 3 or when compared to the wild type EGFR peptide or when compared to an EGFR peptide comprising a wild type Domain IV. In some of these embodiments, the anti-EGFR antibody is cetuximab, matuzumab, necitumumab, panitumumab, zalutumumab, or nimotuzumab. In particular embodiments, the anti-EGFR antibody that binds better to the presently disclosed modified EGFR peptides in comparison to the previously disclosed EGFRt peptide (set forth in SEQ ID NO: 3) is cetuximab. In certain embodiments, an anti-EGFR antibody (e.g., cetuximab) binds to a presently disclosed modified EGFR peptide at least 10%, at least 20%, at least 30%, at least 40%, at least 50%, at least 60%, at least 70%, at least 80%, at least 90%, at least 95%, at least 100%, at least 150% (1.5X) at least 200% (2X), at least 300% (3X) or more when compared to the EGFRt peptide set forth in SEQ ID NO: 3.

In specific embodiments, a nucleic acid molecule is provided that comprises a nucleic acid sequence encoding one of the modified EGFR peptides disclosed herein. In some embodiments, the modified EGFR peptide is expressed in a genetically-modified cell as part of a construct such as, for example, a chimeric antigen receptor construct or an exogenous T cell receptor construct. Accordingly, genetically-modified cells, and populations thereof, are provided comprising the novel modified EGFR peptides disclosed herein, as well as methods of making and isolating the genetically-modified cells comprising the novel modified EGFR peptides. Further disclosed herein are methods of administering a genetically-modified cell comprising a modified EGFR peptide disclosed herein in order to reduce the symptoms or severity of a disease. Administration of genetically-modified cells comprising the modified EGFR peptides disclosed herein may reduce the symptoms or severity of diseases, such as cancers, autoimmune disorders, and other conditions which can be targeted by genetically-modified cells of the present disclosure. Also disclosed herein are methods of immunotherapy for treating cancer in a subject in need thereof comprising administering to the subject a pharmaceutical composition comprising a genetically-modified cell disclosed herein and a pharmaceutically acceptable carrier.

### 2.2 Nucleic Acid Molecules Encoding Modified EGFR Peptides

Provided herein are nucleic acid molecules encoding novel modified EGFR peptides and variants thereof having anti-EGFR antibody binding activity (i.e., active variants). The ability of an antibody to bind to EGFR can be determined using any method known in the art, including but not limited to an enzyme-linked immunosorbent assay (ELISA), immunocytochemistry, immunohistochemistry, flow cytometry, fluorescenceactivated cell sorting (FACS), and immunoprecipitation.

Accordingly, nucleic acid molecules are provided comprising nucleic acid sequences that encode the modified EGFR peptides set forth in SEQ ID NOs: 15-19 and 25, and active variants thereof. Specifically, the nucleic acid sequence set forth in SEQ ID NO: 20 encodes the modified EGFR peptide of SEQ ID NO: 15, referred to herein as the modified EGFR mutant 2 peptide (*i.e.,* T2), and comprises modified EGFR Domain IV mutant 2 (set forth in SEQ ID NO: 5 and encoded by SEQ ID NO: 10), as well as a wild-type EGFR Domain III and wild-type EGFR transmembrane domain.

The nucleic acid sequence set forth in SEQ ID NO: 21 encodes the modified EGFR peptide of SEQ ID NO: 16, referred to herein as the modified EGFR mutant 3 peptide (*i.e*., T3), and comprises modified EGFR Domain IV mutant 3 (set forth in SEQ ID NO: 6 and encoded by SEQ ID NO: 11), as well as a wild-type EGFR Domain III and wild-type EGFR transmembrane domain.

The nucleic acid sequence set forth in SEQ ID NO: 22 encodes the modified EGFR peptide of SEQ ID NO: 17, referred to herein as the modified EGFR mutant 4 peptide (*i.e.*, T4), and comprises modified EGFR Domain IV mutant 4 (set forth in SEQ ID NO: 7 and encoded by SEQ ID NO: 12), as well as a wild-type EGFR Domain III and wild-type EGFR transmembrane domain.

The nucleic acid sequence set forth in SEQ ID NO: 23 encodes the modified EGFR peptide of SEQ ID NO: 18, referred to herein as the modified EGFR mutant 5 peptide (*i.e.*, T5), and comprises modified EGFR Domain IV mutant 5 (set forth in SEQ ID NO: 8 and encoded by SEQ ID NO: 13), as well as a wild-type EGFR Domain III and wild-type EGFR transmembrane domain.

The nucleic acid sequence set forth in SEQ ID NO: 24 encodes the modified EGFR peptide of SEQ ID NO: 19, referred to herein as the modified EGFR mutant 6 peptide (*i.e*., T6), and comprises modified EGFR Domain IV mutant 6 (set forth in SEQ ID NO: 9 and encoded by SEQ ID NO: 14), as well as a wild-type EGFR Domain III and wild-type EGFR transmembrane domain.

The nucleic acid sequence set forth in SEQ ID NO: 26 encodes the modified EGFR peptide of SEQ ID NO: 25, referred to herein as the modified EGFR mutant 7 peptide, which lacks an EGFR Domain IV, and only comprises a wild-type EGFR Domain III and wild-type EGFR transmembrane domain.

Also provided herein are active variants of the nucleic acid sequences encoding the modified EGFR peptides disclosed herein, wherein the variant nucleic acid sequences encode a modified EGFR peptide having the ability to bind to an anti-EGFR antibody (*e.g*., cetuximab). Further provided are variants of the modified EGFR peptides disclosed herein that retain antibody-binding activity.

As used herein, "variants" is intended to mean substantially similar sequences. A "variant" polypeptide is intended to mean a polypeptide derived from the "native" or "wild type" polypeptide by deletion and/or addition of one or more amino acids at one or more internal sites in the native protein and/or substitution of one or more amino acids at one or more sites in the native polypeptide. Likewise, a "variant" polynucleotide is a polynucleotide derived from the "native" or "wild type" polynucleotide by deletion and/or addition of one or more nucleic acids at one or more sites in the native nucleotide sequence. As used herein, a "native" polynucleotide or polypeptide comprises a parental sequence from which variants are derived. The parental nucleic acid sequences of variant polynucleotides encoding modified EGFR peptides may include SEQ ID NOs: 20-24 and 26. Likewise, the parental polypeptide sequences of variant modified EGFR polypeptides may include SEQ ID NOs: 15-19 and 25.

Variant polypeptides disclosed herein are biologically active. That is, they continue to possess the desired biological activity of the native protein; *i.e*., anti-EGFR antibody-binding activity. Such variants may result, for example, from human manipulation. Biologically active variants of a native modified EGFR peptide of the embodiments (*e.g*., SEQ ID NOs: 15-19 and 25), or variants of the native nucleic acid sequences (*e.g*., SEQ ID NOs: 20-24 and 26) encoding the modified EGFR peptides disclosed herein, will have at least about 40%, about 45%, about 50%, about 55%, about 60%, about 65%, about 70%, about 75%, about 80%, about 85%, about 90%, about 91%, about 92%, about 93%, about 94%, about 95%, about 96%, about 97%, about 98%, or about 99%, sequence identity to the amino acid sequence of the native polypeptide or nucleic acid sequence of the native polynucleotide, as determined by sequence alignment programs and parameters described elsewhere herein. A biologically active variant of a modified EGFR peptide of the embodiments may differ from that modified EGFR peptide by as few as about 1-20 amino acid residues, as few as about 1-10, as few as about 1-5, as few as about 4, as few as 3, 2, or even 1 amino acid residue.

The polypeptides of the embodiments may be altered in various ways including amino acid substitutions, deletions, truncations, and insertions. Methods for such manipulations are generally known in the art. For example, amino acid sequence variants can be prepared by mutations in the DNA. Methods for mutagenesis and polynucleotide alterations are well known in the art. See, for example, Kunkel (1985) Proc. Natl. Acad. Sci. USA 82:488-492; Kunkel et al. (1987) Methods in Enzymol. 154:367-382; U.S. Pat. No. 4,873,192; Walker and Gaastra, eds. (1983) Techniques in Molecular Biology (MacMillan Publishing Company, New York) and the references cited therein. Guidance as to appropriate amino acid substitutions that do not affect biological activity of the protein of interest may be found in the model of Dayhoff et al. (1978) Atlas of Protein Sequence and Structure (Natl. Biomed. Res. Found., Washington, D.C.). Conservative substitutions, such as exchanging one amino acid with another having similar properties, may be optimal.

Depending on the context, "fragment" refers to a portion of the amino acid sequence of a polypeptide or protein, or polynucleotide encoding a portion of the amino acid sequence of a polypeptide or protein. Fragments may retain the activity of the original protein and hence, such "active" fragments include, for example, fragments of modified EGFR peptides, such as a fragment of any one of SEQ ID NO: 15-19 and 25 that retains antibody-binding activity. A fragment of a nucleotide sequence encoding a modified EGFR peptide, such as a fragment of any one of SEQ ID NOs: 20-24 and 26 may encode a protein fragment that is biologically active. A biologically active nucleotide fragment can be prepared by isolating a portion of a nucleic acid sequence encoding a modified EGFR peptide, expressing the encoded portion of the modified EGFR peptide, and assessing the activity of the encoded portion of the modified EGFR peptide. Fragments of modified EGFR peptides include fragments of SEQ ID NOS: 15-19 and 25. Fragments of modified EGFR peptides comprise at least about 15, 20, 30, 35, 36, 37, 38, 39, 40, 41, or 42 amino acids.

In certain embodiments, expression cassettes or expression constructs are provided for the expression of at least one modified EGFR peptide disclosed herein, or active variant thereof, in a cell. In some embodiments, the cassette includes 5' and 3' regulatory sequences operably linked to a nucleic acid molecule provided herein encoding a novel modified EGFR peptide, or active variant thereof. "Operably linked" is intended to mean a functional linkage between two or more elements. For example, an operable linkage between a nucleic acid sequence encoding a modified EGFR peptide as disclosed herein and a regulatory sequence (*e.g*., a promoter) is a functional link that allows for expression of the nucleic acid sequence encoding the modified EGFR peptide. Operably linked elements may be contiguous or non-contiguous. When used to refer to the joining of two protein coding regions, by operably linked is intended that the coding regions are in the same reading frame.

In some embodiments, the cassette further comprises at least one additional gene to be co-transformed into a cell. In further embodiments, the additional gene(s) are provided on multiple expression cassettes. In some embodiments, such an expression cassette is provided with a plurality of restriction sites and/or recombination sites for insertion of a recombinant polynucleotide to be under the transcriptional regulation of the regulatory regions.

In some embodiments, the expression cassette includes in the 5'-3' direction of transcription, a transcriptional and translational initiation region (*i.e.,* a promoter), a nucleic acid sequence encoding a modified EGFR peptide, or active variant thereof, as disclosed herein, and a transcriptional and translational termination region (*i.e*., termination region) functional in genetically-modified cells of the present disclosure. The regulatory regions (*i.e*., promoters, transcriptional regulatory regions, and translational termination regions) and/or a nucleic acid molecule provided herein may be native/analogous to the host cell or to each other. Alternatively, the regulatory regions and/or a nucleic acid molecule provided herein may be heterologous to the host cell or to each other. As used herein, "heterologous" in reference to a sequence is a sequence that originates from a foreign species, or, if from the same species, is substantially modified from its native form in composition and/or genomic locus by deliberate human intervention. For example, a promoter operably linked to a heterologous nucleic acid molecule is from a species different from the species from which the nucleic acid molecule was derived, or, if from the same/analogous species, one or both are substantially modified from their original form and/or genomic locus, or the promoter is not the native promoter for the operably linked nucleic acid molecule. Alternatively, the regulatory regions and/or a nucleic acid molecule provided herein may be entirely synthetic.

The termination region may be native with the transcriptional initiation region, may be native with the operably linked nucleic acid molecule, may be native with the cell host, or may be derived from another source (*i.e.*, foreign or heterologous) to the promoter, the nucleic acid molecule, the cell host, or any combination thereof. In preparing the expression cassette, the various DNA fragments may be manipulated, so as to provide for the DNA sequences in the proper orientation. Toward this end, adapters or linkers may be employed to join the DNA fragments or other manipulations may be involved to provide for convenient restriction sites, removal of superfluous DNA, removal of restriction sites, or the like. For this purpose, *in vitro* mutagenesis, primer repair, restriction, annealing, resubstitutions, *e.g*., transitions and transversions, may be involved.

In some embodiments, a number of promoters are used in the expression cassettes provided herein. One example of a suitable promoter is the immediate early cytomegalovirus (CMV) promoter sequence. This promoter sequence is a strong constitutive promoter sequence capable of driving high levels of expression of any polynucleotide sequence operatively linked thereto. Another example of a suitable promoter is Elongation Growth Factor-1α (EF-1α). However, other constitutive promoter sequences may also be used, including, but not limited to the simian virus 40 (SV40) early promoter, mouse mammary tumor virus (MMTV), human immunodeficiency virus (HIV) long terminal repeat (LTR) promoter, MoMuLV promoter, an avian leukemia virus promoter, an Epstein-Barr virus immediate early promoter, a Rous sarcoma virus promoter, as well as human gene promoters such as, but not limited to, the actin promoter, the myosin promoter, the hemoglobin promoter, and the creatine kinase promoter. Further, the present disclosure should not be limited to the use of constitutive promoters. Inducible promoters are also contemplated as part of the present disclosure. The use of an inducible promoter provides a molecular switch capable of turning on expression of the polynucleotide sequence which it is operatively linked when such expression is desired, or turning off the expression when expression is not desired. Examples of inducible promoters include, but are not limited to a metallothionine promoter, a glucocorticoid promoter, a progesterone promoter, and a tetracycline promoter. Synthetic promoters are also contemplated as part of the present disclosure, for example, the JeT promoter (see, WO/2002/012514).

In some embodiments, the promoters are selected based on the desired outcome. It is recognized that different applications can be enhanced by the use of different promoters in the expression cassettes to modulate the timing, location and/or level of expression of the polynucleotides disclosed herein. Such expression constructs may also contain, if desired, a promoter regulatory region (*e.g*., one conferring inducible, constitutive, environmentally- or developmentally-regulated, or cell- or tissue-specific/selective expression), a transcription initiation start site, a ribosome binding site, an RNA processing signal, a transcription termination site, and/or a polyadenylation signal.

In some embodiments, the nucleic acid molecule encoding the presently disclosed modified EGFR peptides further encodes a signal sequence that directs the expression of the modified EGFR to the cell surface. In some of these embodiments, the signal sequence is a native EGFR signal sequence, such as the sequence set forth in amino acid residues 1-24 of SEQ ID NO: 2, or a heterologous signal sequence. In some embodiments, the signal sequence is a signal peptide from any type 1 transmembrane protein (extracellular N-terminus), which allows for the transport of the presently disclosed modified EGFR peptides to the cell membrane and cell surface and allows for the correct localization of the modified EGFR protein, in particular the extracellular portion on the cell surface, and the transmembrane portion inserted into the plasma membrane, and the cytoplasmic portion, if present, in the host cell. In these embodiments, the signal sequence is present on the amino terminus of the modified EGFR peptide and in some of these embodiments, the signal sequence is cleavable to produce the mature modified EGFR peptide (*e.g*., after passage through the endoplasmic reticulum). In particular embodiments, the signal sequence comprises a granulocyte macrophage colony-stimulating factor receptor (GMCSFR) alpha chain signal sequence. In particular embodiments, the GMCSFR alpha chain signal sequence comprises SEQ ID NO: 27.

In specific embodiments, the nucleic acid molecule comprising a nucleotide sequence encoding a modified EGFR peptide further comprises a nucleotide sequence encoding a chimeric antigen receptor (CAR) or an exogenous T cell receptor. An expression cassette which further comprises a nucleotide sequence encoding a CAR is referred to herein as a CAR expression cassette. An expression cassette which further comprises a nucleotide sequence encoding an exogenous TCR is referred to herein as a TCR expression cassette.

In certain embodiments, the CAR expression cassette or TCR expression cassette comprises one or more sequences encoding an extracellular, target-specific binding element otherwise referred to as a ligand-binding domain or moiety. The choice of ligand-binding domain depends upon the type and number of ligands that define the surface of a target cell. For example, the ligand-binding domain may be chosen to recognize a ligand that acts as a cell surface marker on target cells associated with a particular disease state. Thus, examples of cell surface markers that may act as ligands for the ligand-binding domain in the CAR or exogenous TCR can include those associated with viral, bacterial and parasitic infections, autoimmune disease, and cancer cells.

In specific embodiments, the extracellular ligand-binding domain is specific for an antigen of a cancer cell. In the context of the present disclosure, "tumor antigen" refers to antigens that are common to specific hyperproliferative disorders such as cancer. As non-limiting examples, in some embodiments the antigen of the target is a tumor-associated surface antigen, such as ErbB2 (HER2/neu), carcinoembryonic antigen (CEA), epithelial cell adhesion molecule (EpCAM), epidermal growth factor receptor (EGFR), EGFR variant III (EGFRvIII), CD19, CD20, CD30, CD40, disialoganglioside GD2, ductal-epithelial mucine, gp36, TAG-72, glycosphingolipids, glioma-associated antigen, B-human chorionic gonadotropin, alphafetoprotein (AFP), lectin-reactive AFP, thyroglobulin, RAGE-1, MN-CA IX, human telomerase reverse transcriptase, RU1, RU2 (AS), intestinal carboxyl esterase, mut hsp70-2, M-CSF, prostase, prostase specific antigen (PSA), PAP, NY-ESO-1, LAGA-la, p53, prostein, PSMA, surviving and telomerase, prostate-carcinoma tumor antigen-1 (PCTA-1), MAGE, ELF2M, neutrophil elastase, ephrin B2, CD22, insulin growth factor (IGFl)-l, IGF-II, IGFI receptor, mesothelin, a major histocompatibility complex (MHC) molecule presenting a tumor-specific peptide epitope, 5T4, ROR1, Nkp30, NKG2D, tumor stromal antigens, the extra domain A (EDA) and extra domain B (EDB) of fibronectin and the Al domain of tenascin-C (TnC Al) and fibroblast associated protein (fap); a lineage-specific or tissue specific antigen such as CD3, CD4, CD8, CD24, CD25, CD33, CD34, CD133, CD138, CTLA-4, B7- 1 (CD80), B7-2 (CD86), endoglin, a major histocompatibility complex (MHC) molecule, BCMA (CD269, TNFRSF 17), CS 1, or a virus-specific surface antigen such as an HIV-specific antigen (such as HIV gpl20); an EBV-specific antigen, a CMV-specific antigen, a HPV-specific antigen such as the E6 or E7 oncoproteins, a Lasse Virus-specific antigen, an Influenza Virus-specific antigen, as well as any derivate or variant of these surface markers. In a particular embodiment of the present disclosure, the ligand-binding domain is specific for CD19.

In some embodiments, the extracellular domain of a chimeric antigen receptor further comprises an autoantigen (see, Payne et al. (2016) Science, Vol. 353 (6295): 179-184), which can be recognized by autoantigen-specific B cell receptors on B lymphocytes, thus directing T cells to specifically target and kill autoreactive B lymphocytes in antibody-mediated autoimmune diseases. Such CARs can be referred to as chimeric autoantibody receptors (CAARs), and the incorporation of one or more co-stimulatory domains described herein into such CAARs is encompassed by the present disclosure.

The CAR expression cassette encodes an intracellular signaling domain of a CAR, which is responsible for activation of at least one of the normal effector functions of the cell in which the CAR has been placed and/or activation of proliferative and cell survival pathways. The term "effector function" refers to a specialized function of a cell. Effector function of a T cell, for example, may be cytolytic activity or helper activity including the secretion of cytokines. An intracellular signaling domain, such as CD3 ζ, can provide an activation signal to the cell in response to binding of the extracellular domain. As discussed, the activation signal can induce an effector function of the cell such as, for example, cytolytic activity or cytokine secretion.

In some embodiments, the CAR intracellular domain includes one or more intracellular co-stimulatory domains, which transmit a co-stimulatory signal which promotes cell proliferation, cell survival, and/or cytokine secretion after binding of the extracellular domain. In some embodiments, such intracellular co-stimulatory domains can be any of those described in the Examples herein (*i.e.,* N6), and any of those known in the art, such as, for example, a CD28 domain, a 4-1BB domain, an OX-40 domain, an ICOS domain, or a CD27 domain.

In some embodiments, a CAR disclosed herein further comprises a transmembrane domain and hinge region which link the extracellular ligand-binding domain or autoantigen with the intracellular signaling and co-stimulatory domains. In some embodiments, the transmembrane domain and/or hinge region are derived from CD8α.

In a specific embodiment, the CAR expression cassette encodes a CAR comprising an anti-CD19 scFv and a CD3ζ signaling domain. In some of these embodiments, the CAR expression cassette further comprises at least one co-stimulatory domain.

In other specific embodiments, the CAR expression cassette comprises a nucleic acid molecule encoding an inducible regulatory construct which comprises at least one co-stimulatory domain. As used herein, a "regulatory expression cassette" refers to an expression cassette comprising a nucleic acid molecule encoding an inducible regulatory construct. An expression cassette can be both a CAR expression cassette and a regulatory expression cassette.

For example, in some embodiments, expression cassettes comprise sequences encoding a binding domain and at least one co-stimulatory domain, wherein a small molecule, antibody, or other molecule binds to the binding domain to induce dimerization of two inducible regulatory constructs. In some embodiments, such dimerization initiates the co-stimulatory signal to the cell to promote proliferation, survival, and/or cytokine secretion. In some embodiments, wherein the binding domain can bind a small molecule, the binding domain comprises an analogue of FKBP12 (*e.g.*, comprising an F36V substitution) and the small molecule is rimiducid (*i.e.,* AP1903). Any binding domains known in the art to be useful in such inducible regulatory constructs, such as CAR T cell safety switches and the like, are contemplated in the present disclosure.

In certain embodiments, the modified EGFR peptide serves as a selectable marker that allows for the isolation and enrichment of cells that are co-expressing a sequence of interest, such as a CAR or exogenous TCR. In other embodiments, the nucleic acid molecule or expression cassette comprising a nucleotide sequence encoding the presently disclosed modified EGFR peptides can further comprise a selectable marker gene or a reporter gene, or both (other than the modified EGFR peptide itself), to facilitate identification and selection of desired cells from the population of cells sought to be transfected or infected through viral vectors. In other aspects, the selectable marker may be carried on a separate piece of DNA and used in a co-transfection procedure. Both selectable markers and reporter genes may be flanked with appropriate regulatory sequences to enable expression in the host cells. Useful selectable markers include, for example, antibiotic-resistance genes and fluorescent marker genes. In certain embodiments, the selectable marker gene is a CD34 epitope. In some of these embodiments, the CD34 epitope is a QBend10 epitope, which can be recognized by the anti-CD34 QBend10 antibody clone. The epitope of the QBend10 anti-CD34 antibody has been mapped to the extracellular domain of CD34 between amino acids 43 and 49 (Jones *et al.* (1996) in Leukocyte Typing VI). In some embodiments, the QBend10 epitope comprises the amino acid sequence set forth in SEQ ID NO: 28.

In some embodiments, the selectable marker (*e.g*., CD34 epitope) is positioned at the C-terminus or the N-terminus of the EGFR Domain III, at the C-terminus or N-terminus of a modified EGFR Domain IV, or it is positioned within the modified EGFR Domain IV.

In those embodiments wherein the nucleic acid molecule comprising a nucleotide sequence encoding a presently disclosed modified EGFR peptide further comprises at least one additional nucleotide sequence encoding a sequence of interest (*e.g*., encoding a CAR or exogenous TCR), the two or more nucleotide sequences can be transcribed into a single polycistronic mRNA under the control of a single promoter using any element known in the art to allow for the translation of two or more genes from the same mRNA molecule including, but not limited to, an IRES element or a 2A element (*e.g*., a T2A element, a P2A element, an E2A element, and an F2A element). In some of these embodiments, the nucleic acid molecule encoding the modified EGFR peptide can be 5' or 3' to the additional sequence of interest (e.g., a CAR encoding nucleotide sequence or exogenous TCR encoding sequence).

Also provided herein are vectors comprising the nucleic acid molecules encoding the novel modified EGFR peptides of the present disclosure. In some embodiments, vectors comprise a nucleic acid molecule encoding the novel modified EGFR peptides or an expression cassette as disclosed herein. In particular embodiments, the vector further comprises a coding sequence for a CAR or exogenous TCR. In certain embodiments, the vector further comprises a coding sequence for a CD34 epitope. In some embodiments, nucleic acids encoding the modified EGFR peptides disclosed herein (and in some embodiments, a CD34 epitope and/or CAR or exogenous TCR) are cloned into a number of types of vectors. For example, in some embodiments the nucleic acid is cloned into a vector including but not limited to a plasmid, a phagemid, a phage derivative, an animal virus, and a cosmid. Vectors of particular interest include expression vectors, replication vectors, probe generation vectors, and sequencing vectors.

In specific embodiments, nucleic acid molecules encoding a modified EGFR peptide are provided on viral vectors, such as retroviral vectors, lentiviral vectors, adenoviral vectors, and adeno-associated viral (AAV) vectors. Viral vector technology is well known in the art and is described, for example, in Sambrook et al. (2001, Molecular Cloning: A Laboratory Manual, Cold Spring Harbor Laboratory, New York), and in other virology and molecular biology manuals. Viruses, which are useful as vectors include, but are not limited to, retroviruses, adenoviruses, adeno-associated viruses, herpes viruses, and lentiviruses. In general, a suitable vector contains an origin of replication functional in at least one organism, a promoter sequence, convenient restriction endonuclease sites, and one or more selectable markers, (*e.g*., WO 01/96584; WO 01/29058; and U.S. Pat. No. 6,326,193).

### 2.3 Methods for Producing Recombinant Viral Vectors

In some embodiments, the present disclosure provides recombinant AAV vectors for use in the methods of the present disclosure. Recombinant AAV vectors are typically produced in mammalian cell lines such as HEK-293. Because the viral *cap* and *rep* genes are removed from the vector to prevent its self-replication to make room for the therapeutic gene(s) to be delivered (*e.g.* the endonuclease gene), it is necessary to provide these *in trans* in the packaging cell line. In addition, it is necessary to provide the "helper" (*e.g*. adenoviral) components necessary to support replication (Cots D, Bosch A, Chillon M (2013) Curr. Gene Ther. 13(5): 370-81). Frequently, recombinant AAV vectors are produced using a tripletransfection in which a cell line is transfected with a first plasmid encoding the "helper" components, a second plasmid comprising the *cap* and *rep* genes, and a third plasmid comprising the viral ITRs containing the intervening DNA sequence to be packaged into the virus. Viral particles comprising a genome (ITRs and intervening gene(s) of interest) encased in a capsid are then isolated from cells by freeze-thaw cycles, sonication, detergent, or other means known in the art. Particles are then purified using cesium-chloride density gradient centrifugation or affinity chromatography and subsequently delivered to the gene(s) of interest to cells, tissues, or an organism such as a human patient. Accordingly, methods are provided herein for producing recombinant AAV vectors comprising at least one nucleic acid sequence encoding a modified EGFR peptide described herein, such as SEQ ID NOs: 15-19 and 25, or active variants thereof. In some of these embodiments, the recombinant AAV vector further encodes a CD34 antigen and/or a CAR or exogenous TCR.

In some embodiments, genetic transfer is accomplished via lentiviral vectors. Lentiviruses, in contrast to other retroviruses, in some contexts may be used for transducing certain non-dividing cells. Non-limiting examples of lentiviral vectors include those derived from a lentivirus, such as Human Immunodeficiency Virus 1 (HIV-1), HIV-2, an Simian Immunodeficiency Virus (SIV), Human T-lymphotropic virus 1 (HTLV-1), HTLV-2 or equine infection anemia virus (E1AV). For example, lentiviral vectors have been generated by multiply attenuating the HIV virulence genes, for example, the genes env, vif, vpr, vpu and nef are deleted, making the vector safer for therapeutic purposes. Lentiviral vectors are known in the art, see Naldini *et al.,* (1996 and 1998); Zufferey *et al.,* (1997); Dull *et al.,* 1998, U.S. Pat. Nos. 6,013,516; and 5,994,136). In some embodiments, these viral vectors are plasmid-based or virus-based, and are configured to carry the essential sequences for incorporating foreign nucleic acid, for selection, and for transfer of the nucleic acid into a host cell. Known lentiviruses can be readily obtained from depositories or collections such as the American Type Culture Collection ("ATCC"; 10801 University Blvd., Manassas, Va. 20110-2209), or isolated from known sources using commonly available techniques.

In specific embodiments, lentiviral vectors are prepared using a plasmid encoding the gag, pol, tat, and rev genes cloned from human immunodeficiency virus (HIV) and a second plasmid encoding the envelope protein from vesicular stomatitis virus (VSV-G) used to pseudotype viral particles. A transfer vector, such as the pCDH-EF1-MCS vector, can be used with a suitable promoter such as the JeT promoter or the EF1 promoter. Nucleotide sequences encoding the modified EGFR peptides disclosed herein can then be inserted downstream of the promoter, followed by an IRES and GFP. All three plasmids can then be transfected into lentivirus cells, such as the Lenti-X-293T cells, and lentivirus can then be harvested, concentrated and screened after a suitable incubation time. Accordingly, methods are provided herein for producing recombinant lentiviral vectors comprising at least one nucleic acid sequence encoding a modified EGFR peptide described herein, such as SEQ ID NOs: 15-19 and 25, or active variants thereof. In some of these embodiments, the recombinant lentiviral vector further encodes a CD34 antigen and/or a CAR or exogenous TCR.

### 2.4 Genetically-Modified Cells and Populations Thereof Comprising Modified EGFR Peptides

Provided herein are cells genetically-modified to contain at least one modified EGFR peptide, such as any one of SEQ ID NOs: 15-19 and 25, or an active variant thereof, as disclosed herein. In different variations of the present disclosure, a nucleic acid molecule or expression cassette encoding a modified EGFR peptide described herein is present within the genome of the genetically-modified cell or, alternatively, is not integrated into the genome of the cell. In some embodiments where the nucleic acid molecule or expression cassette is not integrated into the genome, the nucleic acid molecule or expression cassette is present in the genetically-modified cell in a recombinant DNA construct, in an mRNA, in a viral genome, or other nucleic acid which is not integrated into the genome of the cell.

In certain embodiments, the genetically-modified cells further express a CAR or an exogenous T cell receptor described herein. In some of these embodiments, the nucleic acid molecule encoding the CAR or exogenous T cell receptor is integrated into the genome of the genetically-modified cell.

In particular embodiments, the genetically-modified cells further express a CD34 epitope, such as a QBend10 epitope described herein. In some of these embodiments, the nucleic acid molecule encoding the CD34 epitope is integrated into the genome of the genetically-modified cell.

In some genetically-modified cells embodied herein, the nucleic acid molecule encoding the modified EGFR peptide (and in some embodiments, also encoding a CD34 epitope and/or a CAR or an exogenous T cell receptor) is positioned with the endogenous T cell receptor alpha gene of the cell. In some of these embodiments, the modified EGFR peptide encoding sequence is positioned within the endogenous T cell receptor alpha constant region gene, such as within exon 1 of the T cell receptor alpha constant region gene, and optionally within an intron 5' upstream of exon 1 of the T cell receptor alpha constant region gene.

In specific embodiments, the cells comprising the modified EGFR peptides are eukaryotic cells. In particular embodiments, the cells comprising the modified EGFR peptides are T cells or NK cells, particularly human T cells or NK cells, or cells derived therefrom. In some embodiments, the cells are primary human T cells or primary human NK cells.

T cells and NK cells can be obtained from a number of sources, including peripheral blood mononuclear cells, bone marrow, lymph node tissue, cord blood, thymus tissue, tissue from a site of infection, ascites, pleural effusion, spleen tissue, and tumors. In certain embodiments of the present disclosure, any number of T cell and NK cell lines available in the art may be used. In some embodiments of the present disclosure, T cells and NK cells are obtained from a unit of blood collected from a subject using any number of techniques known to the skilled artisan. In one embodiment, cells from the circulating blood of an individual are obtained by apheresis.

The invention further provides a population of genetically-modified cells comprising a plurality of a genetically-modified cells comprising in their genome a presently disclosed nucleic acid molecule encoding a modified EGFR peptide described herein. Methods disclosed herein allow for the enrichment of cells expressing the modified EGFR peptide and in some embodiments, co-expressing a CD34 epitope and/or a CAR or exogenous TCR. As used herein, the term "enriching," "enrichment," or any variation thereof, refers to the process of increasing the concentration of target genetically-modified cells within a population of cells to obtain a new population of cells with a higher concentration of the target genetically-modified cells. For example, a heterogeneous population of cells can be enriched to select for those genetically-modified cells expressing a modified EGFR peptide as disclosed herein. In specific embodiments, the genetically-modified cells expressing a modified EGFR peptide further express a CAR, exogenous TCR, and/or a CD34 epitope. Enriching a population of cells for those cells expressing a modified EGFR peptide can be carried out using anti-EGFR antibodies using standard methods known in the art and disclosed elsewhere herein. Enriching a population of cells for those cells expressing a modified EGFR peptide and a CD34 epitope described herein can be carried out using anti-CD34 antibodies using standard methods known in the art and disclosed elsewhere herein.

Thus, in various embodiments of the invention, a population of genetically-modified cells is provided wherein at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 96%, at least 97%, at least 98%, at least 99%, or up to 100%, of cells in the population are a genetically-modified cell expressing a modified EGFR peptide disclosed herein. In certain embodiments, a population of genetically-modified cells is provided wherein at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 96%, at least 97%, at least 98%, at least 99%, or up to 100%, of cells in the population express both a modified EGFR peptide and a CAR or exogenous TCR. In other embodiments, a population of genetically-modified cells is provided wherein at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 96%, at least 97%, at least 98%, at least 99%, or up to 100%, of cells in the population express a modified EGFR peptide, a CD34 epitope, and a CAR or exogenous TCR.

In particular embodiments genetically-modified cells are provided wherein the nucleic acid molecules disclosed herein encoding a modified EGFR peptide are integrated into the TCR alpha gene of the cells. In those embodiments wherein the integrated nucleic acid molecule encoding a modified EGFR peptide is positioned within a TCR alpha gene (e.g., within a TCR alpha constant region gene or within an intron 5' upstream of exon 1 of the T cell receptor alpha constant region gene), a population of genetically-modified cells is provided wherein at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 96%, at least 97%, at least 98%, at least 99%, or up to 100%, of cells in the population express a modified EGFR peptide and do not express an endogenous TCR. In some of these embodiments, a population of genetically-modified cells is provided wherein at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 96%, at least 97%, at least 98%, at least 99%, or up to 100%, of cells in the population express both a modified EGFR peptide and a CAR or exogenous TCR and do not express an endogenous TCR. In other of these embodiments, a population of genetically-modified cells is provided wherein at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 96%, at least 97%, at least 98%, at least 99%, or up to 100%, of cells in the population express a modified EGFR peptide, a CD34 epitope, and a CAR or exogenous TCR, but do not express an endogenous TCR.

A population of cells which is enriched for cells expressing a presently disclosed modified EGFR peptide is also provided. In some of these embodiments, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 96%, at least 97%, at least 98%, at least 99%, or up to 100%, of cells in the population express a modified EGFR peptide (and in some embodiments, a CD34 epitope and/or a CAR or exogenous TCR).

### 2.5 Methods for Producing and Isolating Genetically-Modified Cells

The present disclosure provides methods for producing genetically-modified cells comprising a modified EGFR peptide as disclosed herein (and in some embodiments, a CD34 epitope and/or a CAR or exogenous TCR). In different aspects of the present disclosure, a nucleic acid molecule or expression cassette encoding a modified EGFR peptide disclosed herein, is integrated into the genome of the cell or, alternatively, is not integrated into the genome of the cell. The presently disclosed methods for producing a genetically-modified cell expressing a modified EGFR comprises introducing into a cell a nucleic acid molecule described herein that encodes a modified EGFR peptide, and introducing into the cell either an engineered nuclease protein, or a second nucleic acid molecule encoding an engineered nuclease protein, wherein the engineered nuclease is expressed in the cell. The engineered nuclease recognizes and cleaves a recognition sequence in the genome of the cell to produce a cleavage site and the nucleic acid encoding the modified EGFR is inserted into the genome of the cell at the cleavage site such that the modified EGFR is expressed by the cell.

In some embodiments, DNA or RNA encoding the modified EGFR peptides disclosed herein is introduced into a cell using any technology known in the art. In specific embodiments, vectors or expression cassettes comprising the nucleic acids encoding the modified EGFR peptides disclosed herein is introduced into a cell using a viral vector. Such vectors are known in the art and include lentiviral vectors, adenoviral vectors, and adeno-associated virus (AAV) vectors (reviewed in Vannucci, et al. (2013 New Microbiol. 36:1-22). Recombinant AAV vectors useful in the present disclosure can have any serotype that allows for transduction of the virus into the cell and insertion of the nuclease gene into the cell and, in particular embodiments, into the cell genome. In particular embodiments, recombinant AAV vectors have a serotype of AAV2 or AAV6. Recombinant AAV vectors can also be self-complementary such that they do not require second-strand DNA synthesis in the host cell (McCarty, et al. (2001) Gene Ther. 8: 1248-54).

In some embodiments, nucleic acid molecules or expression cassettes disclosed herein are delivered into a cell in the form of DNA (e.g., circular or linearized plasmid DNA or PCR products) or RNA (*e.g*., an mRNA). In some embodiments wherein the engineered nuclease genes are delivered in DNA form (*e.g.* plasmid) and/or via a viral vector (*e.g.* AAV or lentiviral vector), they are operably linked to a promoter or found on an expression cassette disclosed herein. In some embodiments, the promoter is a viral promoter such as endogenous promoters from the viral vector (*e.g.* the LTR of a lentiviral vector) or the well-known cytomegalovirus- or SV40 virus-early promoters. In other embodiments, the promoter is a synthetic promoter, such as the JeT promoter. In certain embodiments, genes encoding the modified EGFR peptides disclosed herein are operably linked to a promoter that drives gene expression preferentially in the target cell (*e.g.,* a human T cell).

In some embodiments, nucleic acid molecules or expression cassettes encoding modified EGFR peptides disclosed herein, are coupled covalently or non-covalently to a nanoparticle or encapsulated within such a nanoparticle using methods known in the art (Sharma, et al. (2014) Biomed Res Int. 2014). A nanoparticle is a nanoscale delivery system whose length scale is <1 µm, preferably <100 nm. Such nanoparticles may be designed using a core composed of metal, lipid, polymer, or biological macromolecule, and multiple copies of the nucleic acid molecules or expression cassettes can be attached to or encapsulated with the nanoparticle core. This increases the copy number of the DNA that is delivered to each cell and, so, increases the intracellular expression of each engineered nuclease to maximize the likelihood that the modified EGFR peptides will be expressed. The surface of such nanoparticles may be further modified with polymers or lipids (*e.g.,* chitosan, cationic polymers, or cationic lipids) to form a core-shell nanoparticle whose surface confers additional functionalities to enhance cellular delivery and uptake of the payload (Jian et al. (2012) Biomaterials. 33(30): 7621-30). Nanoparticles may additionally be advantageously coupled to targeting molecules to direct the nanoparticle to the appropriate cell type and/or increase the likelihood of cellular uptake. Examples of such targeting molecules include antibodies specific for cell-surface receptors and the natural ligands (or portions of the natural ligands) for cell surface receptors.

In some embodiments, nucleic acid molecules or expression cassettes encoding modified EGFR peptides disclosed herein, are encapsulated within liposomes or complexed using cationic lipids (see, *e.g.,* LIPOFECTAMINE, Life Technologies Corp., Carlsbad, CA; Zuris et al. (2015) Nat Biotechnol. 33: 73-80; Mishra et al. (2011) J Drug Deliv. 2011:863734). The liposome and lipoplex formulations can protect the payload from degradation, and facilitate cellular uptake and delivery efficiency through fusion with and/or disruption of the cellular membranes of the cells.

In some embodiments, nucleic acid molecules or expression cassettes encoding modified EGFR peptides disclosed herein, are encapsulated within polymeric scaffolds (*e.g.,* PLGA) or complexed using cationic polymers (*e.g.,* PEI, PLL) (Tamboli et al. (2011) Ther Deliv. 2(4): 523-536). In some embodiments, nucleic acid molecules or expression cassettes encoding modified EGFR peptides disclosed herein, are combined with amphiphilic molecules that self-assemble into micelles (Tong et al. (2007) J Gene Med. 9(11): 956-66). Polymeric micelles may include a micellar shell formed with a hydrophilic polymer (*e.g.,* polyethyleneglycol) that can prevent aggregation, mask charge interactions, and reduce nonspecific interactions outside of the cell.

In some embodiments, nucleic acid molecules or expression cassettes encoding modified EGFR peptides disclosed herein, are formulated as emulsions for delivery to the cell. The term "emulsion" refers to, without limitation, any oil-in-water, water-in-oil, water-in-oil-in-water, or oil-in-water-in-oil dispersions or droplets, including lipid structures that can form as a result of hydrophobic forces that drive apolar residues (*e.g.,* long hydrocarbon chains) away from water and polar head groups toward water, when a water immiscible phase is mixed with an aqueous phase. These other lipid structures include, but are not limited to, unilamellar, paucilamellar, and multilamellar lipid vesicles, micelles, and lamellar phases. Emulsions are composed of an aqueous phase and a lipophilic phase (typically containing an oil and an organic solvent). Emulsions also frequently contain one or more surfactants. Nanoemulsion formulations are well known, *e.g.,* as described in US Patent Application Nos. 2002/0045667 and 2004/0043041, and US Pat. Nos. 6,015,832, 6,506,803, 6,635,676, and 6,559,189.

In some embodiments, nucleic acid molecules or expression cassettes encoding modified EGFR peptides disclosed herein, are covalently attached to, or non-covalently associated with, multifunctional polymer conjugates, DNA dendrimers, and polymeric dendrimers (Mastorakos et al. (2015) Nanoscale. 7(9): 3845-56; Cheng et al. (2008) J Pharm Sci. 97(1): 123-43). The dendrimer generation can control the payload capacity and size, and can provide a high payload capacity. Moreover, display of multiple surface groups can be leveraged to improve stability and reduce nonspecific interactions.

Methods of introducing and expressing genes into a cell are known in the art. In the context of an expression vector, the vector can be readily introduced into a host cell, *e.g.,* mammalian, bacterial, yeast, or insect cell by any method in the art. For example, the expression vector can be transferred into a host cell by physical, chemical, or biological means. Physical methods for introducing a polynucleotide into a host cell include calcium phosphate precipitation, lipofection, particle bombardment, microinjection, electroporation, and the like. Methods for producing cells comprising vectors and/or exogenous nucleic acids are well-known in the art. See, for example, Sambrook et al. (2001, Molecular Cloning: A Laboratory Manual, Cold Spring Harbor Laboratory, New York). A preferred method for the introduction of a polynucleotide into a host cell is calcium phosphate transfection. Biological methods for introducing a polynucleotide of interest into a host cell include the use of DNA and RNA vectors. Viral vectors, and especially retroviral vectors, have become the most widely used method for inserting genes into mammalian, *e.g.,* human cells. Other viral vectors can be derived from lentivirus, poxviruses, herpes simplex virus I, adenoviruses and adeno-associated viruses, and the like. See, for example, U.S. Pat. Nos. 5,350,674 and 5,585,362. Chemical means for introducing a polynucleotide into a host cell include colloidal dispersion systems, such as macromolecule complexes, nanocapsules, microspheres, beads, and lipid-based systems including oil-in-water emulsions, micelles, mixed micelles, and liposomes. An exemplary colloidal system for use as a delivery vehicle in vitro and *in vivo* is a liposome (e.g., an artificial membrane vesicle).

In some embodiments, the invention further provides for the introduction of the nucleic acid molecules or expression cassettes encoding modified EGFR peptides disclosed herein into the T cell receptor alpha gene. In certain embodiments, the nucleic molecule or expression cassettes are introduced into a recognition sequence present in the T cell receptor alpha constant region gene, which comprises the coding sequences for the T cell receptor alpha subunit. As such, introduction of the nucleic acid molecules or expression cassettes disrupts expression of the endogenous T cell receptor alpha subunit, and consequently disrupts expression of the endogenous T cell receptor. In particular embodiments, such recognition sequences can be present within exon 1 of the T cell receptor alpha constant region gene.

In other particular embodiments, the nucleic acid molecules or expression cassettes are introduced into a recognition sequence within an intron that is positioned 5' upstream of exon 1 of the T cell receptor alpha constant region gene. In some such embodiments, the nucleic acid molecule or expression cassette comprises a 5' homology arm and a 3' homology arm flanking the elements of the insert to promote homologous recombination into a nuclease cleavage site. Such homology arms have sequence homology to corresponding sequences 5' upstream and 3' downstream of the nuclease recognition sequence in the targeted 5' intron where a cleavage site is produced. In general, homology arms can have a length of at least 50 base pairs, preferably at least 100 base pairs, and up to 2000 base pairs or more, and can have at least 90%, preferably at least 95%, or more, sequence homology to their corresponding sequences in the genome. Elements of the insert can include an exogenous splice acceptor site, an IRES or 2A element, a coding sequence for a modified EGFR peptide, and optionally a poly A signal. In various embodiments, the insert comprises, from 5' to 3', an exogenous splice acceptor site, a 2A element or IRES element, a coding sequence for a modified EGFR peptide (and in some embodiments, a CD34 epitope and/or a CAR or exogenous TCR, that can be, in turn, separated by a 2A element or an IRES element), and a polyA signal. In certain embodiments, the 2A element is a T2A, a P2A, an E2A, or an F2A element. In particular embodiments, the 2A element is a T2A element. In specific embodiments, the insert comprises a polyadenylation sequence or polyA signal. Thus, an insert can comprise a poly A signal located 3' downstream of a sequence encoding a modified EGFR peptide (and in some embodiments, a CD34 epitope and/or a CAR or exogenous TCR). In this manner, transcription of the T cell receptor alpha gene, particularly the coding sequences of the T cell receptor alpha constant region locus, will be disrupted by the polyA signal, thus preventing expression of the T cell receptor alpha subunit. In some examples, the exogenous sequence of interest can further include an exogenous branch site positioned 5' upstream of the exogenous splice acceptor site.

Also encompassed within the present invention are methods for isolating a genetically-modified cell that expresses a modified EGFR peptide disclosed herein, wherein the genetically-modified cell disclosed herein is contacted with an anti-EGFR antibody selecting for the genetically-modified cells which are bound to the anti-EGFR antibody. The methods disclosed herein for isolating a genetically-modified cell that expresses a modified EGFR peptide can also be used to enrich a cell population for cells expressing the desired EGFR peptide. Any anti-EGFR antibody that recognizes an epitope on the extracellular domain of EGFR can be used for isolating the genetically-modified cells, including, but not limited to cetuximab, matuzumab, necitumumab, panitumumab, zalutumumab, or nimotuzumab. Any method known in the art for selecting a genetically-modified cell bound to an anti-EGFR antibody can be used for the selection step. Such methods include, but are not limited to fluorescence activated cell sorting (FACS), magnetic activated cell sorting (MACS), and immunoprecipitation. In certain embodiments, the anti-EGFR antibody comprises a detectable label. In some of these embodiments, the detectable label is biotin. In certain embodiments wherein the anti-EGFR antibody is biotinylated, the selection is performed using anti-biotin or streptavidin-coated particles. In some of these embodiments, the particles are magnetic beads and the selection step involves applying a magnetic force to the solution of magnetic beads and cells.

In those embodiments wherein the genetically-modified cells express a CD34 epitope, the method for isolating the genetically-modified cells comprise contacting the genetically-modified cells with an anti-CD34 antibody that recognizes the expressed CD34 epitope and selecting for those genetically-modified cells that are bound to the anti-CD34 antibody. In certain embodiments, the anti-CD34 antibody is conjugated to a particle (e.g., a magnetic bead). In particular embodiments wherein the CD34 epitope is a QBend10 epitope, the anti-CD34 antibody is clone QBend10.

### 2.6 Pharmaceutical Compositions

In some embodiments, the present disclosure provides a pharmaceutical composition comprising a genetically-modified cell, or a population of genetically-modified cells, of the present disclosure expressing a modified EGFR peptide. In particular embodiments, the pharmaceutical composition comprises a genetically-modified cell, or a population of genetically-modified cells, of the present disclosure expressing a modified EGFR peptide and a CAR or exogenous TCR and a pharmaceutically-acceptable carrier (and in some embodiments, a CD34 epitope). Such pharmaceutical compositions can be prepared in accordance with known techniques. See, *e.g.,* Remington, The Science And Practice of Pharmacy (21sted. 2005). In the manufacture of a pharmaceutical formulation according to the present disclosure, cells are typically admixed with a pharmaceutically acceptable carrier and the resulting composition is administered to a subject. The carrier must, of course, be acceptable in the sense of being compatible with any other ingredients in the formulation and must not be deleterious to the subject. In some embodiments, pharmaceutical compositions of the present disclosure further comprise one or more additional agents useful in the treatment of a disease in the subject. In additional embodiments, where the genetically-modified cell is a genetically-modified human T cell or NK cell (or a cell derived therefrom), pharmaceutical compositions of the present disclosure can further include biological molecules, such as cytokines (*e.g.,* IL-2, IL-7, IL-15, and/or IL-21), which promote *in vivo* cell proliferation and engraftment. Pharmaceutical compositions comprising genetically-modified cells of the present disclosure can be administered in the same composition as an additional agent or biological molecule (*e.g.,* a chemotherapeutic agent) or, alternatively, can be co-administered in separate compositions.

The present disclosure also provides genetically-modified cells, or populations thereof, described herein that express a modified EGFR peptide described herein and a CAR or exogenous TCR (and in some embodiments, a CD34 epitope) for use as a medicament. The present disclosure further provides the use of genetically-modified cells or populations thereof described herein in the manufacture of a medicament for treating a disease in a subject in need thereof. In one such aspect, the medicament is useful for cancer immunotherapy in subjects in need thereof.

The present disclosure also provides genetically-modified cells, or populations thereof, described herein that express a modified EGFR peptide described herein and a CAR or exogenous TCR (and in some embodiments, a CD34 epitope) for use in treating a disease in a subject in need thereof. In one such aspect, the CAR or exogenous TCR has specificity for a tumor antigen and is for use in treating cancer in subjects in need thereof.

In some embodiments, the genetically-modified cells, populations thereof, pharmaceutical compositions, and medicaments of the present disclosure are useful for treating any disease state that can be targeted by T cell adoptive immunotherapy. In a particular embodiment, the pharmaceutical compositions of the present disclosure are useful as immunotherapy in the treatment of cancer. Non-limiting examples of cancer which may be treated with the pharmaceutical compositions of the present disclosure are carcinomas, lymphomas, sarcomas, melanomas, blastomas, leukemias, and germ cell tumors, including but not limited to cancers of B-cell origin, neuroblastoma, osteosarcoma, prostate cancer, renal cell carcinoma, rhabdomyosarcoma, liver cancer, gastric cancer, bone cancer, pancreatic cancer, skin cancer, cancer of the head or neck, breast cancer, lung cancer, cutaneous or intraocular malignant melanoma, renal cancer, uterine cancer, ovarian cancer, colorectal cancer, colon cancer, rectal cancer, cancer of the anal region, stomach cancer, testicular cancer, uterine cancer, carcinoma of the fallopian tubes, carcinoma of the endometrium, carcinoma of the cervix, carcinoma of the vagina, carcinoma of the vulva, non-Hodgkin's lymphoma, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, solid tumors of childhood, lymphocytic lymphoma, cancer of the bladder, cancer of the kidney or ureter, carcinoma of the renal pelvis, neoplasm of the central nervous system (CNS), primary CNS lymphoma, tumor angiogenesis, spinal axis tumor, brain stem glioma, pituitary adenoma, Kaposi's sarcoma, epidermoid cancer, squamous cell cancer, environmentally induced cancers including those induced by asbestos, multiple myeloma, Hodgkin lymphoma, non-Hodgkin's lymphomas, acute myeloid lymphoma, chronic myelogenous leukemia, chronic lymphoid leukemia, immunoblastic large cell lymphoma, acute lymphoblastic leukemia, mycosis fungoides, anaplastic large cell lymphoma, and T-cell lymphoma, and any combinations of said cancers. In certain embodiments, cancers of B-cell origin include, without limitation, B-lineage acute lymphoblastic leukemia, B-cell chronic lymphocytic leukemia, B-cell lymphoma, diffuse large B cell lymphoma, pre-B ALL (pediatric indication), mantle cell lymphoma, follicular lymphoma, marginal zone lymphoma, Burkitt's lymphoma, and B-cell non-Hodgkin's lymphoma.

### 2.7 Methods of Administering Genetically-Modified Cells

Also disclosed herein is the administration of the genetically-modified cells or populations thereof of the present disclosure that express a modified EGFR peptide and a CAR or exogenous TCR (and in some embodiments, a CD34 epitope) to a subject in need thereof. The pharmaceutical compositions described herein may be administered to a subject in need thereof. For example, an effective amount of a genetically-modified cell or a population thereof comprising a modified EGFR peptide described herein, and optionally a CAR or exogenous TCR, and further optionally a CD34 epitope, can be administered to a subject having a disease. The disease can be cancer. Thus, the present disclosure also provides a method for providing a T cell-mediated immune response to a target cell population or tissue in a mammal, comprising the step of administering to the mammal a CAR T cell, wherein the CAR comprises an extracellular ligand-binding domain that specifically interacts with a predetermined target, such as a tumor antigen. Unlike antibody therapies, genetically-modified cells of the present disclosure are able to replicate and expand *in vivo,* resulting in long-term persistence that can lead to sustained control of a disease.

Examples of possible routes of administration include parenteral (*e.g.,* intravenous (IV), intramuscular (IM), intradermal, subcutaneous (SC), or infusion) administration. Moreover, the administration may be by continuous infusion or by single or multiple boluses. In specific embodiments, one or both of the agents is infused over a period of less than about 12 hours, 6 hours, 4 hours, 3 hours, 2 hours, or 1 hour. In still other embodiments, the infusion occurs slowly at first and then is increased over time.

In some embodiments, a genetically-modified cell of the present disclosure targets a tumor antigen for the purposes of treating cancer. Such cancers can include, without limitation, carcinomas, adenocarcinomas, lymphomas, sarcomas, melanomas, blastomas, leukemias, and germ cell tumors, including but not limited to cancers of B-cell origin, neuroblastoma, osteosarcoma, prostate cancer, renal cell carcinoma, rhabdomyosarcoma, liver cancer, gastric cancer, bone cancer, pancreatic cancer, skin cancer, cancer of the head or neck, breast cancer, lung cancer, cutaneous or intraocular malignant melanoma, renal cancer, uterine cancer, ovarian cancer, colorectal cancer, colon cancer, rectal cancer, cancer of the anal region, stomach cancer, testicular cancer, uterine cancer, carcinoma of the fallopian tubes, carcinoma of the endometrium, carcinoma of the cervix, carcinoma of the vagina, carcinoma of the vulva, non-Hodgkin's lymphoma, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, solid tumors of childhood, lymphocytic lymphoma, cancer of the bladder, cancer of the kidney or ureter, carcinoma of the renal pelvis, neoplasm of the central nervous system (CNS), primary CNS lymphoma, tumor angiogenesis, spinal axis tumor, brain stem glioma, pituitary adenoma, Kaposi's sarcoma, epidermoid cancer, squamous cell cancer, environmentally induced cancers including those induced by asbestos, multiple myeloma, Hodgkin lymphoma, non-Hodgkin's lymphomas, acute myeloid lymphoma, chronic myelogenous leukemia, chronic lymphoid leukemia, immunoblastic large cell lymphoma, acute lymphoblastic leukemia, mycosis fungoides, anaplastic large cell lymphoma, and T-cell lymphoma, and any combinations of said cancers. In certain embodiments, cancers of B-cell origin include, without limitation, B-lineage acute lymphoblastic leukemia, B-cell chronic lymphocytic leukemia, B-cell lymphoma, diffuse large B cell lymphoma, pre-B ALL (pediatric indication), mantle cell lymphoma, follicular lymphoma, marginal zone lymphoma, Burkitt's lymphoma, and B-cell non-Hodgkin's lymphoma.

When cancer is treated with the presently disclosed genetically-modified cells, the subject administered the genetically-modified cells may further be administered an additional therapeutic, such as radiation, surgery, or a chemotherapeutic agent.

When an "effective amount" or "therapeutic amount" is indicated, the precise amount of the compositions of the present disclosure to be administered can be determined by a physician with consideration of individual differences in age, weight, tumor size (if present), extent of infection or metastasis, and condition of the patient (subject). A pharmaceutical composition comprising the genetically-modified cells described herein may be administered at a dosage of 10⁴ to 10⁹ cells/kg body weight, including all integer values within those ranges. The dosage may be 10⁵ to 10⁶ cells/kg body weight, including all integer values within those ranges. Cell compositions may be administered multiple times at these dosages. The cells can be administered by using infusion techniques that are commonly known in immunotherapy (see, *e.g.,* Rosenberg et al., New Eng. J. of Med. 319:1676, 1988). The optimal dosage and treatment regime for a particular patient can readily be determined by one skilled in the art of medicine by monitoring the patient for signs of disease and adjusting the treatment accordingly.

Administration of genetically-modified cells of the present disclosure may reduce at least one symptom of a target disease or condition. For example, administration of genetically-modified cells of the present disclosure can reduce at least one symptom of a cancer. Symptoms of cancers are well known in the art and can be determined by known techniques.

In certain instances wherein a presently disclosed genetically-modified cell or population thereof is administered to a subject and the subject develops adverse effects, the subject can be administered an anti-EGFR antibody with ADCC or CDC activity, such as but not limited to cetuximab, matuzumab, necitumumab, panitumumab, zalutumumab, and nimotuzumab, in order to reduce the number of circulating cells expressing the modified EGFR peptide. Thus, methods for depleting the presently disclosed genetically-modified cells within a subject are provided, wherein the method involves the administration of an anti-EGFR antibody to the subject. The modified EGFR peptide may function as a suicide gene by serving as a target for the anti-EGFR antibody.

Alternatively, when a depletion of the genetically-modified cells is desired and those genetically-modified cells express a CD34 epitope, an anti-CD34 antibody with ADCC or CDC activity can be administered. When the genetically-modified cell expresses a QBend10 epitope, the anti-CD34 antibody is the QBend10 clone (see, for example, WO 2013153391). *In vivo* depletion of previously administered genetically-modified cells expressing a CAR or exogenous TCR along with the presently disclosed modified EGFR peptides (and in some embodiments, a CD34 epitope) can be especially useful in those instances wherein administration of CAR-expressing cells results in the development of cytokine release syndrome and/or neurotoxicity in the subject.

When an anti-EGFR or anti-CD34 antibody is administered to a subject to deplete the presently disclosed genetically-modified cells, the number of genetically-modified cells within the subject is reduced by at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 96%, at least 97%, at least 98%, at least 99%, or up to 100%.

### EXPERIMENTAL

This disclosure is further illustrated by the following examples, which should not be construed as limiting. Examples not falling within the scope of the claims are for illustrative purposes only

### EXAMPLE 1

### Expression and Characterization of Modified EGFR Peptides

### 1. Purpose

The purpose of this study was to express modified EGFR peptides in human T cells, using targeted insertion of the coding sequences into the T cell receptor alpha constant region gene, and determine their binding characteristics to an anti-EGFR antibody (cetuximab).

### 2. Methods

In the following Examples, wherein a modified EGFR peptide is introduced as a linearized DNA construct, a number of materials are consistently used. Coding sequences for each of the modified human EGFR peptides, as well as coding sequences for reference EGFR peptides, are cloned into a pDI vector, which is linearized by restriction endonuclease digestion prior to nucleofection. In each experiment, human T cells are nucleofected with the linearized pDI vector, an siRNA directed against human TMEM173 (*i.e.,* STING) to reduce cytotoxicity, and mRNA encoding the site-specific TRC 1-2x.87EE meganuclease, which recognizes and cleaves a recognition sequence within exon 1 of the T cell receptor alpha constant region gene.

In each pDI vector, the cassette comprising the coding sequence for the modified EGFR peptide includes a 5' inverted terminal repeat (ITR), a 5' homology arm, a JeT promoter, a coding sequence for an anti-CD 19 CAR, a T2A element, a coding sequence for the modified EGFR peptide, a polyA signal, a 3' homology arm, and a 3' ITR, wherein the homology arms have homology to the sequences upstream and downstream of the TRC 1-2x.87EE recognition sequence. The anti-CD19 CAR comprised an FMC63-based scFv, a CD8 hinge and transmembrane domain, an N6 co-stimulatory domain (developed by Precision BioSciences, Inc.), and a CD3-zeta signaling domain.

In this study, enriched CD3+ T cells from a healthy donor were stimulated with ImmunoCult Human CD3/CD28/CD2 T Cell Activator (StemCell Tech.) and 10ng/mL rhIL-2 (Gibco). All cell culture was carried out in X-VIVO 15 Chemically Defined, Serum-free Hematopoietic Cell Medium (Lonza) supplemented with 5% FBS. After 72 hours of stimulation, samples of 1e6 cells were nucleofected using the Lonza 4D with the following: 1µg TRC1-2x.87EE ARCUS meganuclease mRNA (in-house prep); 1.25µL TMEM 173-1 siRNA (Dharmacon, 100µM); 1µg of one of one of the following linearized DNA fragments:
▪ pDI vector only - no modified EGFR peptide.
▪ pDI D3D4 T2 - encoding a modified T2 EGFR peptide set forth in SEQ ID NO: 15.
▪ pDI D3D4 T3 - encoding a modified T3 EGFR peptide set forth in SEQ ID NO: 16.
▪ pDI D3D4 T4 - encoding a modified T4 EGFR peptide set forth in SEQ ID NO: 17.
▪ pDI D3D4 T5 - encoding a modified T5 EGFR peptide set forth in SEQ ID NO: 18.
▪ pDI D3D4 D3TM - encoding a modified EGFR peptide lacking a Domain IV, as set forth in SEQ ID NO: 25 (not part of the invention).

Cells were expanded for 5 days, then non-edited T cells were depleted using the StemCell Tech CD3 Selection Kit with RapidSpheres, and the EasyPlate magnet, then rested overnight in complete media plus 10ng/mL each of rhIL-15 and rhIL-21(Gibco).

24 hours after CD3 depletion, 3e5 cells were stained with antiCD3-BV711 (BioLegend) and Cetuximab (Abnova) + goat-anti-rabbit AlexaFluor488 (Southern Biotech), then analyzed on the CytoFlex-S cytometer (Beckman Coulter).

Events were gated on lymphocytes using forward and side scatter, then CD3-events were selected to determine EGFR expression.

### 3. Results

Cultures of T cells nucleofected with empty pDI vector, or one of five modified EGFR peptides were evaluated for CD3 expression and for signal intensity using cetuximab (Abnova) and a FITC-conjugated secondary Ab (Figure 2). No EGFR signal was observed in the empty vector control (Figure 2A), nor in the D3TM variant which lacked the entire EGFR Domain IV (Figure 2F). The highest intensity EGFR signal was observed in the T2 EGFR variant (Figure 2B), followed by T3 EGFR (Figure 2C). Signal observed in the T4 EGFR variant was lower still (Figure 2D) while the T5 EGFR variant supported the lowest observable signal (Figure 2E).

### 4. Conclusions

These results indicate that each of the modified EGFR peptides tested was capable of binding cetuximab. Notably, the T2 EGFR and T3 EGFR variants were significantly more readily detected by cetuximab than any of the other variants of our design.

### EXAMPLE 2

### Co-Expression and of Modified EGFR Peptides and Chimeric Antigen Receptors

### 1. Purpose

The purpose of this study was to co-express the modified T2 EGFR and T3 EGFR peptides in human T cells with a chimeric antigen receptor (CAR), and compare their cetuximab-binding characteristics to an EGFR peptide comprising an unmodified wild-type EGFR Domain IV and the wild-type EGFR Domain III.

### 2. Methods

In this study, coding sequences for modified EGFR peptides were incorporated into an expression cassette which comprised a 5' inverted terminal repeat (ITR), a 5' homology arm, a JeT promoter, a coding sequence for an anti-CD 19 CAR previously described, a T2A element, a coding sequence for the modified EGFR peptide, a polyA signal, a 3' homology arm, and a 3' ITR, wherein the homology arms have homology to the sequences upstream and downstream of the TRC 1-2x.87EE recognition sequence. The anti-CD19 CAR incorporated a QBend10 CD34 epitope for CAR detection. These expression cassettes were incorporated into the pDI vector, which was linearized by restriction endonuclease digestion prior to nucleofection.

Similar to Example 1, enriched CD3+ T cells from a healthy donor were stimulated with ImmunoCult Human CD3/CD28/CD2 T Cell Activator (StemCell Tech.) and 10ng/mL rhIL-2 (Gibco). All cell culture was carried out in X-VIVO 15 Chemically Defined, Serum-free Hematopoietic Cell Medium (Lonza) supplemented with 5% FBS. After 72 hours of stimulation, samples of 1e6 cells were nucleofected using the Lonza 4D with the following: 1µg TRC1-2x.87EE ARCUS meganuclease mRNA (in-house prep); 1.25µL TMEM 173-1 siRNA (Dharmacon, 100µM); 1 µg of one of one of the following linearized DNA fragments:
▪ pDI Linear 7002 - CAR; no modified EGFR peptide.
▪ pDI CD34N6 T2 - CAR; modified T2 EGFR peptide set forth in SEQ ID NO: 15.
▪ pDI CD34N6 T3 - CAR; modified T3 EGFR peptide set forth in SEQ ID NO: 16.
▪ pDI CD34N6 D3D4 - CAR; a reference EGFRt polypeptide comprising the wild-type human EGFR transmembrane domain, wild-type Domain IV, and wild-type Domain III, as set forth in SEQ ID NO: 3.

Cells were expanded for 5 days, then non-edited T cells were depleted using the StemCell Tech CD3 Selection Kit with RapidSpheres, and the EasyPlate magnet, then rested overnight in complete media plus 10ng/mL each of rhIL-15 and rhIL-21(Gibco).

24 hours after CD3 depletion, 3e5 cells were stained with Cetuximab (Abnova) + goat-anti-rabbit AlexaFluor488 (Southern Biotech) and antiCD34-PE (Invitrogen), then analyzed on the CytoFlex-S cytometer (Beckman Coulter).

Events were gated on lymphocytes using forward and side scatter, then CD3-events were selected to determine EGFR expression.

### 3. Results

Linearized 7002 did not encode an EGFR variant, but served as a control for targeted insertion of the coding sequence into the TRC 1-2x.87EE cleavage site and CD34 detection (indicating expression of the CAR). Accordingly, a strong CD34 signal was detected, but EGFR signal was not (Figure 3A). In this study, the EGFRt polypeptide (D3D4) described by the `894 publication was included as a point of comparison for EGFR signal, and produced a detectable but low signal. By comparison to the D3D4 variant, both the T2 EGFR and T3 EGFR variants resulted in a significantly increased EGFR signal (Figure 3C and 3D), with the T2 EGFR variant exhibiting the greatest signal observed.

### 4. Conclusions

Our results using the linearized expression systems described in Example 1 and Example 2 indicate that the T2 EGFR and T3 EGFR variants are more readily detected by cetuximab than any of the other variants of our design. In fact, the EGFRt polypeptide (D3D4) described by the `894 publication was not well detected using our targeted insertion platform. While other groups report expression and magnetic separation results using this EGFRt peptide, the use of lentiviruses by these groups to deliver the constructs to T cells suggests that multiple copies may enhance expression levels.

### EXAMPLE 3

### Expression of a CAR/Modified EGFR Construct by AAV

### 1. Purpose

The purpose of this study was to introduce a CAR/modified EGFR T2 coding sequence into a human T cell using an AAV vector to deliver a donor template in combination with a site-specific nuclease. Further, this study characterized AAV-transduced human T cells expressing the CAR and the modified EGFR.

### 2. Methods

In this study, an AAV6 vector was generated which comprised a CAR/EGFR donor template. The cassette comprising the donor template included a 5' inverted terminal repeat (ITR), a 5' homology arm, a JeT promoter, a coding sequence for the anti-CD 19 CAR previously described, a T2A element, a coding sequence for the modified EGFR peptide, a polyA signal, a 3' homology arm, and a 3' ITR, wherein the homology arms have homology to the sequences upstream and downstream of the TRC 1-2x.87EE recognition sequence. The modified EGFR peptide introduced in this study was the T2 EGFR variant set forth in SEQ ID NO: 15. The AAV6 generated is referred to as "N6-T2-AAV6".

Enriched CD3+ T cells from a healthy donor were stimulated with ImmunoCult Human CD3/CD28/CD2 T Cell Activator (StemCell Tech.) and 10ng/mL rhIL-2 (Gibco). All cell culture was carried out in X-VIVO 15 Chemically Defined, Serum-free Hematopoietic Cell Medium (Lonza) supplemented with 5% FBS. After 72 hours of stimulation, two samples of 1e6 cells were nucleofected using the Lonza 4D, treated with 1µg TRC1-2x.87EE ARCUS meganuclease mRNA each.

Cells were then transduced with N6-T2-AAV6 (in-house prep; MOI 50,000). This sample was cultured in serum-free media + 30ng/mL rhIL-2 overnight, then fed with 5% FBS media + rhIL-2 the following day. The TRC-only control was cultured entirely in complete media + rhIL-2.

Research-grade cetuximab was purchased from Invivogen, then labeled with biotin using the EZ-Link Sulfo-NHS-Biotin kit (Thermo Scientific).

Cells from the AAV transduced and TRC-only groups were labeled with the biotinylated cetuximab (1:500), followed by a cocktail of Streptavidin-PE and antiCD3-BV711. Samples were analyzed using the CytoFlex-S (Beckman Coulter).

### 3. Results

As expected, nucleofection with TRC 1-2x.87EE alone did not support labeling by biotinylated cetuximab (Figure 4A), as there was no AAV present to provide the donor template. However, cells nucleofected with TRC 1-2x.87EE, and subsequently transduced with T2-N6-AAV6, stained positive using cetuximab (Figure 4B). As shown, 68.39% of cells stained positive using cetuximab, and 59.79% of cells were CD3-negative and stained positive using cetuximab, indicating that they are T cell receptor negative and express the modified EGFR peptide.

### 4. Conclusions

T2-N6-AAV6 can be used to produce CAR T cells that are detectable using cetuximab. This improves the accuracy with which the CAR+ frequency of an engineered cell product can be measured. Further studies were performed to determine if the signal obtained was sufficient to support magnetic separation of CAR T cells.

### EXAMPLE 4

### Co-Expression and Comparison of Modified EGFR Peptides and Chimeric Antigen Receptors

### 1. Purpose

The purpose of this study was to co-express the modified T2 EGFR (SEQ ID NO: 15) and T3 EGFR (SEQ ID NO: 16) peptides in human T cells with a chimeric antigen receptor (CAR), and compare their cetuximab-binding characteristics to a truncated EGFR peptide comprising wild-type EGFR Domain III and EGFR Domain IV. In this study the CAR/modified EGFR T2, T3, or WT domain (D3/D4) coding sequences were inserted into the genome of human T cells using AAV vectors to deliver a donor template in combination with a site-specific meganuclease. Further, this study characterized AAV-transduced human T cells expressing the CAR and the modified EGFR peptides.

### 2. Methods

In this study, AAV6 vectors were generated which comprised a CAR/EGFR donor template. The cassette comprising the donor template included a 5' inverted terminal repeat (ITR), a 5' homology arm, a coding sequence for the anti-CD 19 CAR previously described, a coding sequence for the modified EGFR peptide, a JeT promoter (two copies, one used on the top strand to drive expression of the CAR, and one on the opposite strand to drive expression of the EGFR peptide), a bi-directional polyA signal between the CAR transgene and EGFR peptide, a 3' homology arm, and a 3' ITR, wherein the homology arms have homology to the sequences upstream and downstream of the TRC 1-2 recognition sequence. The modified EGFR peptides introduced in this study were:
▪ modified T2 EGFR peptide set forth in SEQ ID NO: 15.
▪ modified T3 EGFR peptide set forth in SEQ ID NO: 16.
▪ a reference EGFRt polypeptide (EGFR-D3D4) comprising the wild-type human EGFR transmembrane domain, wild-type Domain IV, and wild-type Domain III, as set forth in SEQ ID NO: 3.

Enriched CD3+ T cells from a healthy donor were stimulated with ImmunoCult Human CD3/CD28/CD2 T Cell Activator (StemCell Tech.) and 10ng/mL rhIL-2 (Gibco). All cell culture was carried out in X-VIVO 15 Chemically Defined, Serum-free Hematopoietic Cell Medium (Lonza) supplemented with 5% FBS. After 72 hours of stimulation, samples of 1e6 cells were nucleofected using the Lonza 4D, treated with 1µg TRC1-2x.87EE ARCUS meganuclease mRNA each.

Cells were then transduced with AAV6-CAR-EGFRD3D4, AAV6-CAR-EGFRt2, or AAV6-CAR-EGFRt3 (in-house preparations; MOI 25,000). This sample was cultured in serum-free media + 30ng/mL rhIL-2 overnight, then fed with 5% FBS media + rhIL-2 the following day. The TRC-only control was cultured entirely in complete media + rhIL-2.

Research-grade cetuximab was purchased from Abnova, and detected with a species-specific secondary antibody conjugated to AlexaFluor488 (Jackson ImmunoResearch).

Cells from the AAV transduced and TRC-only groups were labeled with cetuximab (1:500), followed by a cocktail of AlexaFluor488-conjugated secondary Ab, antiCD3-PE (BioLegend clone UCHT1), and anti-FMC63-Alx647 (BioLegend custom product - clone VM16). Samples were analyzed using the CytoFlex-LX (Beckman Coulter).

### 3. Results

Figure 5 shows histograms illustrating the EGFR signal on non-edited cell populations (closed histograms) and the EGFR signal on corresponding CD3-/CAR+ cell populations (open histograms) from the same sample. The truncated EGFR (EGFR-D3D4) peptide having a wild-type transmembrane domain and wild-type Domain III and Domain IV (set forth in SEQ ID NO: 3), was detected on the surface of CD3-/CAR+ cells with a mean intensity that exceeded background (i.e., non-edited cells) by over 30-fold (Figure 5A).

By contrast, detection of both the EGFRt2 (SEQ ID NO: 15) and EGFRt3 (SEQ ID NO: 16) peptides on CD3-/CAR+ cells were superior to detection of the EGFR-D3D4 peptide, with levels that were approximately 55-fold over background (*i.e.,* non-edited cells; Figure 5B and Figure 5C). In the absence of AAV transduction, cetuximab binding was not detected (Figure 5D).

### 4. Conclusions

In agreement with our results using the linearized expression systems described in Example 1 and Example 2, the T2 EGFR and T3 EGFR variants are more readily detected by cetuximab on CD3-/CAR+ cells than the EGFR-D3D4 peptide (SEQ ID NO: 3), described by the '894 publication. This result, and the degree of superiority of the T2 and T3 peptides, was surprising considering that the T2 and T3 peptides are the most truncated forms of Domain IV that were tested, and it was not expected that a greater difference from the wild-type domain would produce a more beneficial effect.

### EXAMPLE 5

### Enrichment of CAR T Cells Expressing a Modified EGFR Peptide

### 1. Purpose

The purpose of this study was to evaluate different methods for enrichment of CAR T cells expressing a modified EGFR peptide encompassed by the invention. Enrichment was performed by positive selection for EGFR-positive cells or negative selection for CD3-negative cells.

### 2. Methods

Human CD3+ cells transduced with T2-N6-AAV6 were labeled with biotinylated cetuximab (mAb from Invivogen, EZ-Link Sulfo-NHS-Biotin kit from Thermo Scientific), then processed through the EasySep Human Biotin Positive Selection Kit (StemCell Technologies) according to manufacturer protocol. The positive and negative fractions from the isolation procedure were cultured overnight in complete media, supplemented with 10ng/mL each of rhII,-15 and rhIL-21 (Gibco).

The following day, the cell fractions were counted and stained for purity, along with a sample of pre-separation CAR T cells. Cells were labeled with cetuximab (not biotinylated), followed by a cocktail containing antiCD3-BV711 (BioLegend) and goat-anti-rabbit-AlexaFluor 488 (Southern Biotech), then analyzed on the CytoFlex-S (Beckman Coulter).

The remaining EGFR+ cells were then processed through the EasySep CD3 Selection Kit (StemCell Technologies) using the manufacturer's protocol. Again, each fraction was cultured overnight in complete media + 10ng/mL each of rhIL-15 and rhIL-21, then stained for purity the following day with the same cocktail as above.

### 3. Results

The frequency of the desired population (CD3- EGFR+) cells in the starting culture was 51% (Figure 6A). The first magnetic enrichment step, an EGFR positive selection using cetuximab, resulted in a purity of approximately 99% EGFR+. 13.6% of these cells were not edited by TRC1-2x87EE (the CD3+ events - Figure 6B). A considerable number of EGFR+ cells were not recovered from the negative fraction (Figure 6C).

Subjecting the EGFR+ cells (Figure 7A) to a CD3 depletion procedure resulted in a product that exhibited greater than 97% purity for the desired phenotype (CD3- EGFR+ Figure 7B). The retained fraction from the CD3 depletion is displayed in Figure 7C.

### 4. Conclusions

Using biotin-based magnetic enrichment strategies targeting EGFR and CD3 on consecutive days, a highly pure population of T cell receptor-negative, CAR-positive cells can be generated from a highly heterogeneous starting population.

### EXAMPLE 6

### Insertion of a CD34 Epitope Into a Modified EGFR Peptide

### 1. Purpose

The purpose of this experiment was determine if a CD34 epitope could be incorporated into a modified EGFR peptide such that the epitope could be detected without interfering with the binding of an anti-EGFR antibody.

### 2. Methods

pDI vectors were constructed wherein the CD34 epitope (SEQ ID NO: 28) recognized by the QBend10 antibody was inserted into the T2 EGFR variant coding sequence. Two variants were produced. In a first variant identified as "EGFRT2 CD34a", the QBend10 epitope was positioned at the N-terminus of the EGFR Domain III. In a second variant identified as "EGFRT2 CD34b", an additional arginine residue was positioned 5' upstream of the QBend10 epitope and a glycine residue was positioned between the QBend10 epitope and the N-terminus of the EGFR Domain III. Each of the 2 variants were incorporated into expression cassettes comprising a 5' inverted terminal repeat (ITR), a 5' homology arm, a JeT promoter, a coding sequence for an anti-CD 19 CAR previously described, a T2A element, a coding sequence for the modified EGFR and QBend10 epitope, a polyA signal, a 3' homology arm, and a 3' ITR, wherein the homology arms have homology to the sequences upstream and downstream of the TRC 1-2x.87EE recognition sequence.

Each pDI vector was linearized and nucleofected into cultures of activated T cells as described in Examples above, along with TMEM173 siRNA and the TRC 1-2x.87EE meganuclease. Cells were cultured as previously described, and were analyzed on day 5 post-nucleofection. Analyses for cell-surface expression of CD34 and EGFRT2 were carried out using the QBend10 antibody and cetuximab as previously described.

### 3. Results

No signal was observed in a mock control transfected with an empty pDI vector (Figure 8A). However, a CD34 signal was observed in both EGFRT2-CD34 variants. The two variants differed somewhat in their signal intensities for EGFR staining, with EGFRT2 CD34a exhibiting the highest EGFR signal (Figure 8B) and EGFRT2 CD34b exhibiting a slightly dimmer signal (Figure 8C). All EGFR+ events also stained positive for CD34, but there were CD34+ events that did not stain positive for EGFR.

### 4. Conclusions

Inserting the QBend10 epitope into the T2 EGFR variant coding sequence allows for detection of this protein with either the anti-EGFR antibody or the anti-CD34 antibody. The ability to detect CAR T cells with either an anti-CD34 antibody such as QBend10, or an anti-EGFR antibody such as cetuximab, may provide flexibility with respect to CAR T evaluation and enrichment.

## Claims

1. A nucleic acid molecule comprising a nucleotide sequence encoding a modified human epidermal growth factor receptor (EGFR), wherein:
(a) said modified EGFR comprises an EGFR Domain III, a modified EGFR Domain IV, and an EGFR transmembrane domain, wherein said modified EGFR Domain IV is a truncated EGFR Domain IV;
(b) said modified EGFR does not comprise an EGFR Domain I, an EGFR Domain II, an EGFR juxtamembrane domain, or an EGFR tyrosine kinase domain; and
(c) said modified EGFR binds to an anti-EGFR antibody.

2. The nucleic acid molecule of claim 1, wherein said modified EGFR Domain IV comprises an amino acid sequence having at least 90% sequence identity to any one of SEQ ID NOs: 5-9, preferably wherein said modified EGFR Domain IV comprises an amino acid sequence of any one of SEQ ID NOs: 5-9.

3. The nucleic acid molecule of claim 1 or claim 2, wherein said modified EGFR comprises an amino acid sequence having at least 90% sequence identity to any one of SEQ ID NOs: 15-19, preferably wherein said modified EGFR comprises an amino acid sequence of any one of SEQ ID NOs: 15-19.

4. The nucleic acid molecule of any one of claims 1-3, wherein said nucleic acid molecule further encodes a signal sequence which directs expression of said modified EGFR to the cell surface, preferably wherein said signal sequence is a GMCSFR alpha chain signal sequence, more preferably wherein said GMCSFR alpha chain signal sequence comprises SEQ ID NO: 27.

5. The nucleic acid molecule of any one of claims 1-4, wherein said nucleic acid molecule further comprises a nucleotide sequence encoding a chimeric antigen receptor or an exogenous T cell receptor.

6. The nucleic acid molecule of any one of claims 1-5, wherein said nucleic acid molecule further comprises a promoter which drives expression of said chimeric antigen receptor or said exogenous T cell receptor, and drives expression of said modified EGFR.

7. The nucleic acid molecule of any one of claims 1-6, wherein said nucleic acid molecule is an mRNA, a recombinant DNA construct, or a viral genome of a viral vector.

8. The nucleic acid molecule of any one of claims 1-7, wherein said nucleic acid molecule further comprises a nucleotide sequence encoding a CD34 epitope.

9. A recombinant DNA construct comprising said nucleic acid molecule of any one of claims 1-8.

10. A viral vector comprising said nucleic acid molecule of any one of claims 1-8, preferably wherein said viral vector is a recombinant adeno-associated viral (AAV) vector.

11. A genetically-modified cell comprising in its genome said nucleic acid molecule of any one of claims 1-8, wherein said genetically-modified cell expresses said modified EGFR, preferably wherein said genetically-modified cell is a genetically-modified human T cell, or a cell derived therefrom.

12. A pharmaceutical composition comprising a pharmaceutically-acceptable carrier and said genetically-modified cell of claim 11.

13. A method for isolating a genetically-modified cell which expresses said modified EGFR of any one of claims 1-8, said method comprising:
(a) contacting said genetically-modified cell of claim 11 with an anti-EGFR antibody; and
(b) selecting said genetically-modified cells which are bound to said anti-EGFR antibody.

14. The genetically modified cell of claim 11 for use in treating a disease in a subject in need thereof.

## Patentansprüche

1. Ein Nukleinsäuremolekül umfassend eine Nukleotidsequenz, die einen modifizierten humanen epidermalen Wachstumsfaktor-Rezeptor (EGFR) kodiert, wobei:
(a) jener modifizierte EGFR eine EGFR-Domäne III, eine modifizierte EGFR-Domäne IV und eine EGFR-Transmembrandomäne umfasst, wobei jene modifizierte EGFR-Domäne IV eine verkürzte EGFR-Domäne IV ist;
(b) jener modifizierte EGFR eine EGFR-Domäne I, eine EGFR-Domäne II, eine EGFR-Juxtamembrandomäne oder eine EGFR-Tyrosinkinasedomäne nicht umfasst; und
(c) jener modifizierte EGFR an einen anti-EGFR-Antikörper bindet.

2. Das Nukleinsäuremolekül aus Anspruch 1, wobei jene modifizierte EGFR-Domäne IV eine Aminosäuresequenz mit mindestens 90% Sequenzidentität zu irgendeinem von SEQ ID NOs: 5-9 umfasst, vorzugsweise wobei jene modifizierte EGFR-Domäne IV eine Aminosäuresequenz von irgendeinem von SEQ ID NOs: 5-9 umfasst.

3. Das Nukleinsäuremolekül aus Anspruch 1 oder Anspruch 2, wobei jener modifizierte EGFR eine Aminosäuresequenz mit mindestens 90% Sequenzidentität zu irgendeinem von SEQ ID NOs: 15-19 umfasst, vorzugsweise wobei jener modifizierte EGFR eine Aminosäuresequenz von irgendeinem von SEQ ID NOs: 15-19 umfasst.

4. Das Nukleinsäuremolekül aus irgendeinem der Ansprüche 1-3, wobei jenes Nukleinsäuremolekül weiter eine Signalsequenz kodiert, die die Expression jenes modifizierten EGFR an die Zelloberfläche leitet, vorzugsweise wobei jene Signalsequenz eine GMCSFR-Alphaketten-Signalsequenz ist, bevorzugter wobei jene GMCSFR-Alphaketten-Signalsequenz SEQ ID NO: 27 umfasst.

5. Das Nukleinsäuremolekül aus irgendeinem der Ansprüche 1-4, wobei jenes Nukleinsäuremolekül weiter eine Nukleotidsequenz umfasst, die einen chimären Antigenrezeptor oder einen exogenen T-Zell-Rezeptor kodiert.

6. Das Nukleinsäuremolekül aus irgendeinem der Ansprüche 1-5, wobei jenes Nukleinsäuremolekül weiter einen Promotor umfasst, der die Expression jenes chimären Antigenrezeptors oder jenes exogenen T-Zell-Rezeptors steuert, und die Expression jenes modifizierten EGFR steuert.

7. Das Nukleinsäuremolekül aus irgendeinem der Ansprüche 1-6, wobei jenes Nukleinsäuremolekül eine mRNA, ein rekombinantes DNA-Konstrukt oder ein virales Genom eines viralen Vektors ist.

8. Das Nukleinsäuremolekül aus irgendeinem der Ansprüche 1-7, wobei jenes Nukleinsäuremolekül weiter eine Nukleotidsequenz umfasst, die ein CD34-Epitop kodiert.

9. Ein rekombinantes DNA-Konstrukt umfassend jenes Nukleinsäuremolekül aus irgendeinem der Ansprüche 1-8.

10. Ein viraler Vektor umfassend jenes Nukleinsäuremolekül aus irgendeinem der Ansprüche 1-8, vorzugsweise wobei jener virale Vektor ein rekombinanter Adeno-assoziierter viraler (AAV) Vektor ist.

11. Eine genetisch modifizierte Zelle umfassend in ihrem Genom jenes Nukleinsäuremolekül aus irgendeinem der Ansprüche 1-8, wobei jene genetisch modifizierte Zelle jenen modifizierten EGFR exprimiert, vorzugsweise wobei jene genetisch modifizierte Zelle eine genetisch modifizierte humane T-Zelle oder eine davon abgeleitete Zelle ist.

12. Eine pharmazeutische Zusammensetzung umfassend einen pharmazeutisch annehmbaren Träger und jene genetisch modifizierte Zelle aus Anspruch 11.

13. Ein Verfahren zum Isolieren einer genetisch modifizierten Zelle, die jenen modifizierten EGFR aus irgendeinem der Ansprüche 1-8 exprimiert, jenes Verfahren umfassend:
(a) Kontaktieren jener genetisch modifizierten Zelle aus Anspruch 11 mit einem anti-EGFR-Antikörper; und
(b) Selektieren jener genetisch modifizierten Zellen, die an jenen anti-EGFR-Antikörper gebunden sind.

14. Die genetisch modifizierte Zelle aus Anspruch 11 zur Verwendung in der Behandlung einer Krankheit in einem Subjekt, das dessen bedarf.

## Revendications

1. Molécule d'acide nucléique comprenant une séquence nucléotidique codant pour un récepteur de facteur de croissance épidermique humain (EGFR) modifié, dans laquelle :
(a) ledit EGFR modifié comprend un domaine III d'EGFR, un domaine IV d'EGFR modifié, et un domaine transmembranaire d'EGFR, dans laquelle ledit domaine IV d'EGFR modifié est un domaine IV d'EGFR tronqué ;
(b) ledit EGFR modifié ne comprend pas un domaine I d'EGFR, un domaine II d'EGFR, un domaine juxtamembranaire d'EGFR, ou un domaine tyrosine kinase d'EGFR ; et
(c) ledit EGFR modifié se lie à un anticorps anti-EGFR.

2. Molécule d'acide nucléique selon la revendication 1, dans laquelle ledit domaine IV d'EGFR modifié comprend une séquence d'acides aminés présentant au moins 90 % d'identité de séquence avec l'une quelconque parmi les SEQ ID NO : 5 à 9, de préférence dans laquelle ledit domaine IV d'EGFR modifié comprend une séquence d'acides aminés de l'une quelconque parmi les SEQ ID NO : 5 à 9.

3. Molécule d'acide nucléique selon la revendication 1 ou la revendication 2, dans laquelle ledit EGFR modifié comprend une séquence d'acides aminés présentant au moins 90 % d'identité de séquence avec l'une quelconque parmi les SEQ ID NO : 15 à 19, de préférence dans laquelle ledit EGFR modifié comprend une séquence d'acides aminés de l'une quelconque parmi les SEQ ID NO : 15 à 19.

4. Molécule d'acide nucléique selon l'une quelconque des revendications 1 à 3, dans laquelle ladite molécule d'acide nucléique code en outre pour une séquence signal qui dirige l'expression dudit EGFR modifié vers la surface cellulaire, de préférence dans laquelle ladite séquence signal est une séquence signal de chaîne alpha de GMCSFR, de manière davantage préférée dans laquelle ladite séquence signal de chaîne alpha de GMCSFR comprend SEQ ID NO : 27.

5. Molécule d'acide nucléique selon l'une quelconque des revendications 1 à 4, dans laquelle ladite molécule d'acide nucléique comprend en outre une séquence nucléotidique codant pour un récepteur d'antigène chimère ou un récepteur de cellule T exogène.

6. Molécule d'acide nucléique selon l'une quelconque des revendications 1 à 5, dans laquelle ladite molécule d'acide nucléique comprend en outre un promoteur qui commande l'expression dudit récepteur d'antigène chimère ou dudit récepteur de cellule T exogène, et commande l'expression dudit EGFR modifié.

7. Molécule d'acide nucléique selon l'une quelconque des revendications 1 à 6, dans laquelle ladite molécule d'acide nucléique est un ARNm, une construction d'ADN recombinant, ou un génome viral d'un vecteur viral.

8. Molécule d'acide nucléique selon l'une quelconque des revendications 1 à 7, dans laquelle ladite molécule d'acide nucléique comprend en outre une séquence nucléotidique codant pour un épitope CD34.

9. Construction d'ADN recombinant comprenant ladite molécule d'acide nucléique selon l'une quelconque des revendications 1 à 8.

10. Vecteur viral comprenant ladite molécule d'acide nucléique selon l'une quelconque des revendications 1 à 8, de préférence dans lequel ledit vecteur viral est un vecteur viral adéno-associé (AAV) recombinant.

11. Cellule génétiquement modifiée comprenant dans son génome ladite molécule d'acide nucléique selon l'une quelconque des revendications 1 à 8, dans laquelle ladite cellule génétiquement modifiée exprime ledit EGFR modifié, de préférence dans laquelle ladite cellule génétiquement modifiée est une cellule T humaine génétiquement modifiée, ou une cellule dérivée de celle-ci.

12. Composition pharmaceutique comprenant un support pharmaceutiquement acceptable et ladite cellule génétiquement modifiée selon la revendication 11.

13. Procédé pour isoler une cellule génétiquement modifiée qui exprime ledit EGFR modifié selon l'une quelconque des revendications 1 à 8, ledit procédé comprenant :
(a) la mise en contact de ladite cellule génétiquement modifiée selon la revendication 11 avec un anticorps anti-EGFR ; et
(b) la sélection desdites cellules génétiquement modifiées qui sont liées audit anticorps anti-EGFR.

14. Cellule génétiquement modifiée selon la revendication 11 pour une utilisation dans le traitement d'une maladie chez un sujet en ayant besoin.
